# EUROPEAN PATENT SPECIFICATION

(11) **EP 3 923 936 B1**
(45) Date of publication and mention of the grant of the patent: **09.08.2023**
(21) Application number: 20705786.0
(22) Date of filing: 07.02.2020
(51) Int. Cl.: A61K 31/4184, A61K 31/138, A61K 31/15, A61K 31/37, A61K 31/404, A61K 31/496, A61K 31/522, A61P 1/16, A61P 15/00, A61P 17/06, A61P 19/04, A61P 19/06, A61P 29/00, A61P 31/00, A61P 33/00, A61P 35/00

(54) **PHARMACEUTICAL COMBINATIONS COMPRISING MEBENDAZOLE AND A STRONG OR MODERATE CYP1A2 INHIBITOR**
PHARMAZEUTISCHE KOMBINATIONEN MIT MEBENDAZOL UND EINEM STARKEN ODER MODERATEN CYP1A2-INHIBITOR
ASSOCIATIONS PHARMACEUTIQUES COMPRENANT DU MÉBENDAZOLE ET UN INHIBITEUR FORT OU MODÉRÉ DE LA CYP1A2

(30) Priority: 13.02.2019 GB 201901989
(43) Date of publication of application: 22.12.2021
(73) Proprietor: Zephapharm Ltd, London W1W 6XB (GB)
(72) Inventor: TAYLOR, John, London W1W 6XB (GB)
(74) Representative: J A Kemp LLP
(86) International application number: PCT/GB2020/050287
(87) International publication number: WO 2020/165559

(56) References cited:
- WO-A1-2018/063472
- NOEMI COWAN ET AL: "In Vitro and In Vivo Drug Interaction Study of Two Lead Combinations, Oxantel Pamoate plus Albendazole and Albendazole plus Mebendazole, for the Treatment of Soil-Transmitted Helminthiasis", ANTIMICROBIAL AGENTS AND CHEMOTHERAPY, vol. 60, no. 10, 1 August 2016 (2016-08-01), pages 6127-6133, XP55684230, US ISSN: 0066-4804, DOI: 10.1128/AAC.01217-16
- Shikiya K ET AL: "Treatment of strongyloidiasis with mebendazole and its combination with thiabendazole", PUBMED - Kansenshogaku Zasshi., 1 November 1990 (1990-11-01), pages 1-2, XP055684257, Retrieved from the Internet: URL:https://www.ncbi.nlm.nih.gov/pubmed/22 86784 [retrieved on 2020-04-08]
- NN JISIEIKE-ONUIGBO ET AL: "Armillifer armillatus infection", NIGERIAN JOURNAL OF CLINICAL PRACTICE, vol. 14, no. 4, 1 January 2011 (2011-01-01), pages 501-503, XP55678825, ISSN: 1119-3077, DOI: 10.4103/1119-3077.91767
- MSAGATI T A M ET AL: "Comparative study of sample preparation methods; supported liquid membrane and solid phase extraction in the determination of benzimidazole anthelmintics in biological matrices by liquid chromatography-electrospray-mass spectrometry", TALANTA, ELSEVIER, AMSTERDAM, NL, vol. 69, no. 1, 15 March 2006 (2006-03-15) , pages 243-250, XP025000676, ISSN: 0039-9140, DOI: 10.1016/J.TALANTA.2005.10.002 [retrieved on 2006-03-15]
- PANTZIARKA P ET AL: "Repurposing Drugs in Oncology (ReDO) - Mebendazole as an anti-cancer agent", ECANCERMEDICALSCIENCE, CANCER INTELLIGENCE, vol. 8, no. 1, 10 July 2014 (2014-07-10), XP002779489, ISSN: 1754-6605
- SAVLIK ET AL: "Modulation of porcine biotransformation enzymes by anthelmintic therapy with fenbendazole and flubendazole", RESEARCH IN VETERINARY SCIENCE, BRITISH VETERINARY ASSOCIATION, LONDON, GB, vol. 80, no. 3, 1 June 2006 (2006-06-01), pages 267-274, XP005276512, ISSN: 0034-5288, DOI: 10.1016/J.RVSC.2005.06.006
- Baliharová V ET AL: "The effects of mebendazole on P4501A activity in rat hepatocytes and HepG2 cells. Comparison with tiabendazole and omeprazole", The Journal of pharmacy and pharmacology, 1 January 2003 (2003-01-01), pages 773-781, XP093024609, DOI: 10.1211/0022357021044 Retrieved from the Internet: URL:https://pubmed.ncbi.nlm.nih.gov/128419 37/ [retrieved on 2023-02-16]
- LUNA S. JOFFE ET AL: "The Anti-helminthic Compound Mebendazole Has Multiple Antifungal Effects against Cryptococcus neoformans", FRONTIERS IN MICROBIOLOGY, vol. 8, 28 March 2017 (2017-03-28), XP055606103, DOI: 10.3389/fmicb.2017.00535
- SOTO HORTENSIA ET AL: "Effects of mebendazole on protein biosynthesis and secretion in human-derived fibroblast cultures", BIOCHEMICAL PHARMACOLOGY, vol. 52, no. 2, 1 July 1996 (1996-07-01), pages 289-299, XP093024758, US ISSN: 0006-2952, DOI: 10.1016/0006-2952(96)00207-9 Retrieved from the Internet: URL:https://pubmed.ncbi.nlm.nih.gov/869485 4/>
- TARA WILLIAMSON ET AL: "Mebendazole and a non-steroidal anti-inflammatory combine to reduce tumor initiation in a colon cancer preclinical model", ONCOTARGET, vol. 7, no. 42, 18 October 2016 (2016-10-18), XP055527194, DOI: 10.18632/oncotarget.11851
- Anonymous: "Guidance Document: Notes on the Design of Bioequivalence study: Mebendazole", WHO/PQT: medicines, 29 March 2021 (2021-03-29), pages 1-2, XP093024761, Retrieved from the Internet: URL:https://extranet.who.int/pqweb/sites/d efault/files/documents/BE_Mebendazole_Marc h2021.pdf [retrieved on 2023-02-16]
- Anonymous: "Drug Interactions & Labelling - Drug Development and Drug Interactions: Table of Substrates, Inhibitors and Inducers", , 26 January 2023 (2023-01-26), pages 1-17, XP093024762, Retrieved from the Internet: URL:https://www.fda.gov/drugs/drug-interac tions-labeling/drug-development-and-drug-i nteractions-table-substrates-inhibitors-an d-inducers#classInhibit [retrieved on 2023-02-16]
- Anonymous: "Guideline on the investigation of drug interactions", , 21 June 2012 (2012-06-21), pages 2-59, XP055727049, Internet Retrieved from the Internet: URL:https://www.ema.europa.eu/en/documents /scientific-guideline/guideline-investigat ion-drug-interactions-revision-1_en.pdf [retrieved on 2020-09-02]

## Description

### Field of the invention

The present invention relates to pharmaceutical combinations comprising the benzimidazole compound mebendazole, and a strong or moderate cytochrome P450 1A2 isoenzyme (CYP1A2) inhibitor for use in a method of treatment of cancer, a non-cancer proliferative disease, a fungal disease, an inflammatory disease, or a fibrotic disease in a human. The present invention also relates to pharmaceutical combinations comprising the benzimidazole compound mebendazole, and the CYP1A2 inhibitor fluvoxamine.

### Background to the invention

Benzimidazole compounds represent a class of pharmacologically active compounds which are known to have activity at biological targets associated with the treatment of a number of diseases. Benzimidazoles such as mebendazole, nocodazole, benomyl, carbendazim, oxifendazole (oxfendazole), albendazole, ricobendazole (albendazole sulphoxide), thiabendazole, fenbendazole, triclabendazole and flubendazole are known to have antiparasitic including anthelminthic and antiprotozoal activity and act as microtubule inhibitors. These microtubule inhibiting benzimidazoles have activity in animal cells as well as in parasites. Benzimidazole compounds have also been found to have additional mechanisms of action which are relevant to all types of cancer / tumours and other proliferative diseases.

As mentioned, mebendazole is a benzimidazole compound. Mebendazole (IUPAC name: methyl *N-*(6-benzoyl-1*H*-benzimidazol-2-yl)carbamate) has been shown to have potent anti-proliferative activity by microtubule inhibition in mammalian cells including tumour cells, as well as in parasites. It also has additional mechanisms of action and antifungal activity. Various studies of mebendazole and its medicinal properties have been carried out.

Dayan, Acta Tropica., 2003, 86, 141 is a review of the pharmacokinetics and toxicity of albendazole, mebendazole and praziquantel.

Dawson et al., British Journal of Clinical Pharmacology, 1982, 14, 453 relates to the pharmacokinetics and bioavailability of mebendazole in man.

Dawson et al., British Journal of Clinical Pharmacology, 1985, 19, 79 relates to the pharmacokinetics and bioavailability of a tracer dose of [3H]-mebendazole in man.

Cowan et al., International Journal for Parasitology: Drugs and Drug Resistance, 2016, 6, 159, relates to the exposure of *Heligmosomoides polygyrus* and *Trichuris muris* to albendazole, albendazole sulfoxide, mebendazole and oxantel pamoate *in vitro* and in *vivo* to elucidate the pathway of drug entry into these gastrointestinal nematodes.

WO 2016/127168 relates to the mebendazole polymorph for treatment and prevention of tumors.

Luder et al., European Journal of Clinical Pharmacology, 1986, 31, 443 relates to the treatment of hydatid disease with high oral doses of mebendazole.

Pawluk et al., Clinical Pharmacokinetics, 2015, 54, 371 is a review of pharmacokinetic drug-drug interactions with the anthelmintic medications albendazole and mebendazole.

Bekhti et al., British Journal of Clinical Pharmacology, 1987, 24, 390 considers how cimetidine increases serum mebendazole concentrations.

Murray et al., Xenobiotica, 2001, 31, 135 relates to the inhibition of human CYP1A2 activity in *vitro* by methylxanthines: potent competitive inhibition by 8-phenyltheophylline.

The academic dissertation by Karjalainen, Department of Clinical Pharmacology University of Helsinki, 2008, relates to the inhibition of CYP1A2-mediated drug metabolism *in vitro* and in humans

Zhou et al., Drug Metabolism Reviews, 2009, 41, 89 relates to polymorphism of human cytochrome P450 (CYP) enzymes and its clinical impact.

Braithwaite et al., European Journal of Clinical Pharmacology, 1982, 22, 161, relates to clinical pharmacokinetics of high dose mebendazole in patients treated for cystic hydatid disease.

Luder et al., European Journal of Clinical Pharmacology, 1986, 31, 443, relates to the treatment of hydatid disease with high oral doses of mebendazole.

El-Khouly et al., Frontiers in Oncology, 2017, 7, 1 relates to effective drug delivery in diffuse intrinsic pontine glioma and the physicochemical properties of mebendazole.

Joffe et al. Frontiers in Microbiology, 2017, 8, 535 relates to the antifungal effects of mebendazole against Cryptococcus neoformans.

Pantziarka et al., Ecancermedicalscience, 2014, 8, 443 discuss mebendazole as an anti-cancer agent in the context of repurposing drugs in oncology.

Baliharova et al., J Pharm Pharmacol., 2003, 55, 773 discuss the effects of mebendazole on P4501A activity in rat hepatocytes and HepG2 cells.

Low systemic bioavailability of mebendazole has limited the development of clinical treatments against proliferative diseases including different types of cancers as well as against parasites. In particular, mebendazole has been considered primarily to be insufficiently bioavailable due to being too poorly absorbed via the gastrointestinal tract (followed by first-pass metabolism of absorbed mebendazole in the liver) to be suitable for treatments which require systemic distribution of therapeutic concentrations of the active drug, mebendazole, e.g. proliferative diseases including different cancers as well as many parasitic and fungal diseases.

### Summary of the invention

The references to methods of treatment in this description are to be interpreted as references to the compounds, pharmaceutical compositions and medicaments of the present invention for use in a method for treatment of the human body by therapy (or for diagnosis).

In addition, the references to compositions and combinations in this description are to be interpreted as references to the compositions and combinations for use in the methods of treatment defined herein (with the exception of references to compositions and combinations as defined in claims 14 and 15).

It has now been found that the systemic bioavailability of the benzimidazole compound, mebendazole, is improved by administration in combination with a strong or moderate CYP1A2 inhibitor.

Accordingly, in a first aspect, the present invention provides a pharmaceutical composition comprising mebendazole or a pharmaceutically acceptable salt, solvate, hydrate, or N-oxide thereof and a strong or moderate cytochrome P450 1A2 isoenzyme (CYP1A2) inhibitor for use in a method of treatment of cancer, a non-cancer proliferative disease, a fungal disease, an inflammatory disease, or a fibrotic disease in a human.

In some embodiments, the CYP1A2 inhibitor is furafylline, ciprofloxacin, enoxacin, fluvoxamine, zafirlukast, 8-phenyltheophylline, methoxsalen, thiabendazole or mexiletine, or a pharmaceutically acceptable salt, solvate, hydrate or N-oxide thereof.

In an aspect, the present invention provides mebendazole or a pharmaceutically acceptable salt, solvate, hydrate, or N-oxide thereof for use in a method of treatment of cancer, a non-cancer proliferative disease, a fungal disease, inflammatory disease, or a fibrotic disease in a human, which method of treatment comprises the simultaneous, concurrent, separate or sequential administration of a strong or moderate CYP1A2 inhibitor.

In an aspect, the present invention provides a pharmaceutical composition comprising mebendazole or a pharmaceutically acceptable salt, solvate, hydrate, or *N-*oxide thereof and fluvoxamine, or a pharmaceutically acceptable salt, solvate, hydrate, or *N*-oxide thereof.

In an aspect, the present invention provides mebendazole or a pharmaceutically acceptable salt, solvate, hydrate, or *N*-oxide thereof in combination with fluvoxamine, or a pharmaceutically acceptable salt, solvate, hydrate, or N-oxide thereof for use in a method of treatment of a human by therapy, which method comprises the simultaneous, concurrent, separate or sequential administration of mebendazole or a pharmaceutically acceptable salt, solvate, hydrate, or N oxide thereof and fluvoxamine, or a pharmaceutically acceptable salt, solvate, hydrate, or *N*-oxide thereof.

Furthermore, it has now been found that poor absorption via the gastrointestinal tract may not be the primary factor limiting systemic bioavailability of mebendazole, and that limited systemic bioavailability may be due to metabolism by CYP1A2. The aspects of the invention enable improved systemic bioavailability of mebendazole by combining it with a strong or moderate CYP1A2 inhibitor. The present invention therefore provides a means of enabling systemic exposure to mebendazole having anti-proliferative including anti-cancer activity, anti-parasitic activity and anti-fungal activity.

### Brief description of the drawings

Figure 1 shows the stability of mebendazole in the presence of CYP1A2 recombinant P450 isoform of Example 3.
Figure 2 shows stability of mebendazole, in human liver microsomes in the absence of a CYP1A2 inhibitor of Example 4.
Figures 3 to 5 show stability of mebendazole, in human liver microsomes in the presence of 3 µM, 10 µM, and 30 µM furafylline (strong CYP1A2 inhibitor) of Example 4.
Figures 6 to 8 show stability of mebendazole, in human liver microsomes in the presence of 30 µM, 100 µM, and 300 µM cimetidine (weak CYP1A2 inhibitor) of Example 4.
Figure 9 shows stability of mebendazole, in human hepatocytes in the absence of a CYP1A2 inhibitor of Example 5.
Figures 10 to 12 show stability of mebendazole, in human hepatocytes in the presence of 3 µM, 10 µM, and 30 µM furafylline (strong CYP1A2 inhibitor) of Example 5.
Figures 13 to 15 show stability of mebendazole, in human hepatocytes in the presence of 30 µM, 100 µM, and 300 µM cimetidine (weak CYP1A2 inhibitor) of Example 5.
Figure 16 shows stability of mebendazole, in human hepatocytes in the absence of a CYP1A2 inhibitor of Example 6.
Figures 17 to 19 show stability of mebendazole, in human hepatocytes in the presence of 10 µM, 30 µM, and 100 µM thiabendazole (moderate CYP1A2 inhibitor) of Example 6.
Figures 20 to 22 show stability of mebendazole, in human hepatocytes in the presence of 30 µM, too µM, and 300 µM cimetidine (weak CYP1A2 inhibitor) of Example 6.
Figure 23 shows stability of mebendazole, in human hepatocytes in the absence of a CYP1A2 inhibitor of Example 7.
Figures 24 to 27 show stability of mebendazole, in human hepatocytes in the presence of 10 µM, 30 µM, 100 µM, and 300 µM fluvoxamine (strong CYP1A2 inhibitor) of Example 7.
Figures 28 to 31 show stability of mebendazole, in human hepatocytes in the presence of 10 µM, 30 µM, 100 µM, and 300 µM mexiletine (moderate CYP1A2 inhibitor) of Example 7.
Figures 32 and 33 show the mebendazole concentration-response curves for 8 neuroblastoma and 13 sarcoma cell lines of Example 9.
Figures 34 and 35 show the mebendazole concentration-response curves for 6 ovarian cancer cell lines and 6 renal cancer cell lines of Example 10.

### Detailed description of the invention

Certain features of the invention are described in more detail below. In the following description, the aspects of the invention are typically referred to collectively as the combinations of the invention.

In the following description, the term "active ingredient" or "active ingredients" of the combinations of the invention refers to mebendazole and the strong or moderate CYP1A2 inhibitor.

The active ingredients of the combinations of the invention may contain asymmetric or chiral centres, and therefore exist in different stereoisomeric forms. Unless otherwise specified, it is intended that all stereoisomeric forms of the active ingredients, including diastereomers, enantiomers and atropisomers, as well as mixtures thereof such as racemic mixtures, may form part of the combinations of the invention. Unless otherwise specified, active ingredients containing one or more chiral centre may be used in enantiomerically or diastereoisomerically pure form, or in the form of a mixture of isomers. If an active ingredient is referred to by name (e.g. by INN or drug trade name), and if that name is generally recognised in the pharmaceutical art as referring to one specific diastereomer, enantiomer or atropisomer, then the active ingredient referred to is typically that specific diastereomer, enantiomer or atropisomer.

Active ingredients of the combinations of the invention may exist in different tautomeric forms, and unless otherwise specified all such forms are embraced within the scope of the invention. The term "tautomer" or "tautomeric form" refers to structural isomers of different energies which are interconvertible via a low energy barrier. For example, proton tautomers (also known as prototropic tautomers) include interconversions via migration of a proton, such as keto- enol tautomerizations. Valence tautomers include interconversions by reorganization of some of the bonding electrons.

Active ingredients of the combinations of the invention in the solid state may exist in different amorphous or crystalline forms (e.g. polymorphic forms), and unless otherwise specified all such forms are embraced within the scope of the invention.

As used herein, a pharmaceutically acceptable salt of an active ingredient refers to a salt of an active ingredient which may be prepared by combining an active ingredient with a base or acid which is acceptable for administration to a human or animal subject.

As used herein, a pharmaceutically acceptable solvate of an active ingredient refers to a solid state complex comprising an active ingredient and molecules of a solvent which is acceptable for administration to a human or animal subject. The active ingredient may be dissolved in the solvent to form a solution or it may be suspended in the solvent to form a suspension. The solution or suspension may be for use as a medicament.

As used herein, a pharmaceutically acceptable hydrate of an active ingredient refers to a solvate in which the solvent is water.

As used herein, a pharmaceutically acceptable *N*-oxide of an active ingredient refers to an oxide of an active ingredient, which active ingredient in its unoxidised state comprises a basic amine or imine.

As used herein, the term "benzimidazole compound" refers to a pharmacologically-active compound comprising a benzimidazole moiety. Mebendazole of the combinations of the present invention typically has anti-proliferative activity including anti-cancer activity, and/or anti-parasitic activity, and/or anti-fungal activity.

Mebendazole, including pharmaceutically acceptable salts, solvates, hydrates, and N-oxides thereof, is used in the combinations of the invention

Mebendazole has now been shown to be metabolised by CYP1A2 using recombinant human P₄₅₀ enzymes, human liver microsome studies, and human hepatocyte studies. "Metabolised by CYP1A2" means that CYP1A2 is responsible at least in part for the metabolism of mebendazole *in vivo.*

In some embodiments of the combinations of the present invention, mebendazole has activity via one or more mechanisms including but not limited to the following:
- microtubule inhibition
- inhibition of cell proliferation
- inhibition of angiogenesis
- inhibition of TRAF2- and NCK-interacting kinase (TNIK)
- interference with vascular epidermal growth factor receptor 2 (VEGFR2)
- induction of apoptosis
- inhibition of metalloproteinases
- activity at BCR-ABL kinase
- activity at BRAF kinase
- Hedgehog signalling pathway inhibition
- induction of proinflammatory (M1) phenotype of monocytoid cells
- antifungal activity, including anticryptococcal activity

Some possible mechanisms are suggested for example in Doudican et al., Anticancer Drugs, 2013, 24, 181; Nygren et al., Acta Oncol, 2013, 52, 427; Pantziarka et al., Ecancermedicalscience, 2014, 8, 485; Tan et al., Scientific Reports, 2016, 6, 33534; Blom et al., Immunopharmacol Immunotoxicol, 2017, 39, 199; De Witt et al., Mol Med, 2017, 23, 50; and Popovic et al., Tropical Journal of Pharmaceutical Research, 2017, 16, 2555.

In some embodiments of the combinations of the present invention, mebendazole has an IC₅₀ of 1 µM or less against a neuroblastoma, sarcoma, renal cancer or ovarian cancer-derived cell lines in cell viability assays.

Mebendazole has been approved worldwide for over four decades (e.g. since 1974 in the USA, and since 1977 in UK) for use as a broad spectrum gastrointestinal anthelminthic drug, with the originator tradename VERMOX^{®} (Janssen Inc.).

Mebendazole potently inhibits proliferating cells, as demonstrated using various tumor cell lines. The long-established mechanism of action of mebendazole is microtubule inhibition by the binding of animal including mammalian cellular tubulin, helminth and protozoal tubulin.

The anti-parasitic action of mebendazole is due to its action as a microtubule-disrupting agent acting to prevent the polymerisation of tubulin, causing parasites to die. Tubulin is vital to cell division and is therefore a cancer target for several widely used chemotherapy drugs, including paclitaxel, colchicine, and vincristine. The inhibition of tubulin polymerisation by mebendazole has been confirmed for example *in vitro* in a glioblastoma model and in a melanoma model. The latter work suggested that the apoptotic response to microtubule disruption is mediated by Bcl-2 phosphorylation. Subsequent work on melanoma confirmed this result, and also showed that mebendazole decreased the levels of X-linked inhibitor of apoptosis (XIAP).

Other proposed mechanisms of action of mebendazole include TRAF2- and NCK-interacting kinase (TNIK) inhibition, anti-angiogenic activity by interference with vascular endothelial growth factor receptor 2 (VEGFR2), and inhibition of metalloproteinases activity.

Mebendazole appears to be effective through p53-dependent and independent pathways. For example, in lung cancer cell lines, it was found that mebendazole treatment caused post-translational p53 stabilization and the downstream expression of p21 and MDM2. In p53-null lung cancer cells, exposure to mebendazole caused cytochrome c accumulation, activation of caspase-9 and caspase-8, and cleavage of PARP and procaspase-3. This independence of p53 status is also evident in the analysis of melanoma cells, where wild-type and mutant p53 cell lines were sensitive to mebendazole.

Mebendazole has been shown to have an anti-angiogenic effect on human lung cancer xenograft models.

Mebendazole was found to interact with several protein kinases including BCR-ABL and BRAF. Analysis of the diagnosis-specific activity of mebendazole showed activity in 80% of the colon cancer cell lines in the NCI 60 panel. Three additional colon cancer cell lines and three cell models with non-malignant phenotypes were subsequently tested, confirming selective colon cancer activity of mebendazole.

Mebendazole has now been shown also to have potent *in vitro* anticancer activity in a wide range of neuroblastoma, sarcoma, ovarian tumor, renal tumor including P-gp expressing multidrug-resistant cell lines, based on studies reported in the examples hereinbelow.

However, systemic bioavailability of mebendazole is low and/or variable in subjects with high inter-patient and intra-patient variability of concentrations of mebendazole observed and, until now, it has not been possible to effectively develop mebendazole as a clinically effective anti-cancer drug or as a drug for the treatment of proliferative non-cancer diseases or as a drug for the effective treatment or more effective treatment of systemic anti-parasitic diseases, or as an anti-fungal treatment.

Mebendazole is commercially available or can be prepared by known methods or by analogy with known methods. For example, mebendazole is available from commercial suppliers including Sigma-Aldrich^{®}.

As described hereinbelow, mebendazole has been found to have high passive permeability, is not a P-glycoprotein (P-gp) substrate, is not a Breast Cancer Resistance Protein (BCRP) substrate, and also is not an Organic Anion Transporting Polypeptide 1B1 (OATP1B1) substrate or Organic Anion Transporting Polypeptide 1B3 (OATP1B3) substrate.

The inventor has now surprisingly found that mebendazole is extensively metabolised by CYP1A2 and its metabolism in human liver microsomes and human hepatocytes is markedly inhibited by moderate and strong CYP1A2 inhibitors.

For this invention, additional investigations have been performed with the benzimidazole compound, ricobendazole (albendazole sulphoxide). Surprisingly, the inventor has found that, as shown in Example 8 hereinbelow, unlike mebendazole, the benzimidazole compound ricobendazole was not metabolised extensively (unlike mebendazole) by the CYP1A2 isoform, and there was zero to only moderate inhibition of intrinsic clearance observed with the specific/selective and strong CYP1A2 inhibitor, furafylline contrasting markedly with the strong (>80%) CYP1A2 inhibition of intrinsic clearance of mebendazole by furafylline as summarized hereinbelow.

### CYP1A2 inhibitor

The CYP1A2 inhibitor of the combinations of the invention is described in more detail below. The following description is applicable to the CYP1A2 inhibitor of each of the first to ninth aspects of the invention.

The CYP1A2 inhibitor is a moderate CYP1A2 inhibitor or a strong CYP1A2 inhibitor. In some embodiments, the CYP1A2 inhibitor is a moderate CYP1A2 inhibitor. In some embodiments, the CYP1A2 inhibitor is a strong CYP1A2 inhibitor. Preferably, the CYP1A2 inhibitor is a strong CYP1A2 inhibitor.

In studies now performed and summarised hereinbelow, all moderate or strong inhibitors of CYP1A2 tested have shown moderate or strong inhibition of the metabolism of mebendazole in human hepatocytes *ex vivo.*

As used herein, a moderate CYP1A2 inhibitor is a substance which is capable in this CYP1A2 metabolic pathway of effecting an inhibition of intrinsic clearance (CLᵢₙₜ) of mebendazole (hereinbelow now established as a CYP1A2 substrate) in human hepatocytes *ex vivo* of 50% to ≤80%, and in this CYP1A2 metabolic pathway would be expected to increase the area under the curve (AUC) *in vivo* of mebendazole by two-fold or more and less than five-fold. Other sensitive index CYP1A2 substrates include, for example, caffeine, theophylline and tizanidine. Examples of moderate CYP1A2 inhibitors include thiabendazole, mexiletine and methoxsalen.

Mexiletine and thiabendazole have now been used in human hepatocytes *ex vivo* studies as moderate CYP1A2 inhibitors. As shown hereinbelow, mexiletine and thiabendazole are moderate to strong CYP1A2 inhibitors of mebendazole metabolism, confirming mebendazole as a sensitive substrate for these CYP1A2 inhibitors in the studies performed.

According to drug labelling, mexiletine is a CYP1A2 inhibitor.

Thiabendazole has been approved as an anthelminthic drug for human use and is currently used as a veterinary (animal use) anthelminthic drug. Thiabendazole has shown anti-tumor activity in vivo, and has vascular disrupting properties (Cha et al., Nat Med, 2012, 18, 1754). Thiabendazole has an inhibition constant (Ki) value for CYP1A2 inhibition of 1.54 µM and an IC50 value for CYP1A2 inhibition of 0.83 µM (Bapiro, Eur J Clin Pharmacol., 2005, 61, 755; Thelingwani et al., Drug Metab Dispos., 2009, 37, 1286).

As used herein, a strong CYP1A2 inhibitor is a substance which is capable in this CYP1A2 metabolic pathway of effecting an inhibition of intrinsic clearance (CLᵢₙₜ) of mebendazole (hereinbelow established as a CYP1A2 substrate) in human hepatocytes *ex vivo* of ≥80%, and in this CYP1A2 metabolic pathway would be expected to increase the AUC in *vivo* of mebendazole by five-fold or more.

Other sensitive index CYP1A2 substrates include caffeine, theophylline and tizanidine. Examples of strong CYP1A2 inhibitors include xanthine derivatives such as furafylline and 8-phenyltheophylline, and ciprofloxacin, enoxacin, fluvoxamine, zafirlukast.

Furafylline and fluxoxamine have now been used in human hepatocytes *ex vivo* studies as strong CYP1A2 inhibitors. As shown hereinbelow, furafylline and fluxoxamine are strong CYP1A2 inhibitors of mebendazole metabolism, confirming mebendazole as a sensitive substrate for these CYP1A2 inhibitors in the studies performed.

Furafylline has now been used as a strong CYP1A2 inhibitor of mebendazole metabolism in human hepatocytes and human liver microsomes *ex vivo* using the weak CYP1A2 inhibitor, cimetidine, as comparator.

Furafylline is a non-competitive inhibitor of CYP1A2 inhibition and has IC50 values of 0.027 µM and 0.07 µM in human liver microsomes for CYP1A2 inhibition of phenacetin-O-deethylase activity (Sesardic et al, British Journal of Clinical Pharmacology, 1990, 29, 651; Obach et al., Drug Metabolism and Disposition, 2007, 35, 246). Furafylline has been shown to be a selective time-dependent inhibitor of CYP1A2. Fluvoxamine has now also been used as a strong CYP1A2 inhibitor of mebendazole metabolism in human hepatocytes *ex vivo.* Fluvoxamine has previously been shown to potently inhibit CYP1A2 phenacetin O-deethylation using human liver microsomes with IC50 values for CYP1A2 inhibition of 0.035 µM and 0.029 µM and Ki of 0.011 µM (Obach et al., Drug Metabolism and Disposition, 2006, 34, 246; and Karjalainen et al., Basic & Clinical Pharmacology & Toxicology, 2008,103,157). According to drug labelling, fluvoxamine is a strong CYP1A2 inhibitor *in vitro* and *in vivo.*

Any compound, which is capable in this CYP1A2 metabolic pathway of effecting an inhibition of intrinsic clearance (CLᵢₙₜ) in human hepatocytes *ex vivo* of mebendazole (hereinbelow established as a CYP1A2 substrate) of ≥50% (e.g. ≥80%), and would be expected in this CYP1A2 metabolic pathway to increase the AUC of mebendazole in *vivo* by 2-fold or more (e.g. 5-fold or more), is considered to be a strong or moderate CYP1A2 inhibitor and can be used in the combinations of the present invention. The strong or moderate CYP1A2 inhibitor may, for example, be a compound which is known to have strong or moderate CYP1A2 inhibitory activity. However the combinations of the invention are not limited thereto and the strong or moderate CYP1A2 inhibitor may be a known substance which is not currently known to be a strong or moderate CYP1A2 inhibitor, or a novel compound which is a strong or moderate CYP1A2 inhibitor.

Moderate and strong CYP1A2 inhibitors are discussed in Drug Interactions & Labelling - Drug Development and Drug Interactions: Table of Substrates, Inhibitors and Inducers, published by the United States Food and Drug Administration (US FDA) and in Guideline CPMP/EWP/560/95/Rev. 1 Corr. 2, Committee for Human Medicinal Products (CHMP), published by the European Medicines Agency (EMA).

Mexiletine is classed as a moderate clinical inhibitor of CYP1A2, fluvoxamine is classed as a strong clinical inhibitor of CYP1A2, and furafylline is classed as a selective time-dependent inhibitor of CYP1A2 in Drug Interactions & Labelling - Drug Development and Drug Interactions: Table of Substrates, Inhibitors and Inducers, published by the United States Food and Drug Administration (US FDA). As used herein, a weak CYP1A2 inhibitor is a substance which is capable in this CYP1A2 metabolic pathway of effecting an inhibition of intrinsic clearance (CLᵢₙₜ) in human hepatocytes *ex vivo* of mebendazole (hereinbelow established as a CYP1A2 substrate) of ≤50%, and would be expected in this CYP1A2 metabolic pathway to increase the AUC plasma value of mebendazole *in vivo* by less than two-fold. It follows from the definitions of strong, moderate and weak CYP1A2 inhibitors above that the strong or moderate CYP1A2 inhibitor in the combinations of the invention is not a weak CYP1A2 inhibitor, or a salt, solvate, hydrate, *N*-oxide, prodrug or active metabolite thereof. Weak CYP1A2 inhibitors include cimetidine, and are discussed in Drug Interactions & Labelling - Drug Development and Drug Interactions: Table of Substrates, Inhibitors and Inducers, published by the United States Food and Drug Administration (US FDA) and in Guideline CPMP/EWP/560/95 /Rev. 1 Corr. 2, Committee for Human Medicinal Products (CHMP), published by the European Medicines Agency (EMA).

Cimetidine is classed as a weak inhibitor of CYP1A2, and has been used in comparative *ex vivo* human hepatocyte studies, hereinbelow summarized, as comparator/reference weak CYP1A2 inhibitor for the moderate and strong CYP1A2 inhibitors, which have been tested.

CYP1A2 inhibition of mebendazole metabolism by cimetidine, as shown hereinbelow, does not occur to any significant extent in human hepatocytes *ex vivo* (i.e. weak inhibition) in contrast to all of the moderate and strong CYP1A2 inhibitors tested, as shown in the new data presented.

Strong or moderate CYP1A2 inhibitors which can be used in the combinations of the invention include xanthine derivatives such as furafylline and 8-phenyltheophylline, ciprofloxacin, enoxacin, fluvoxamine, zafirlukast, methoxsalen, and mexiletine, and pharmaceutically acceptable salts, solvates, hydrates or N-oxides thereof.

Preferred CYP1A2 inhibitors are strong CYP1A2 inhibitors including furafylline, fluvoxamine, thiabendazole (shown hereinbelow to be a strong inhibitor of mebendazole metabolism), 8-phenyltheophylline, ciprofloxacin, enoxacin, and zafirlukast, and pharmaceutically acceptable salts, solvates, hydrates or N-oxides thereof.

Furafylline is a preferred CYP1A2 inhibitor in the combinations of the present invention.

Furafylline is a methylxanthine derivative in clinical development in the mid-1980s, which was originally intended to be developed for the treatment of respiratory diseases. Furafylline is a potent time-dependent inhibitor of CYP1A2. Furafylline has now been shown to be a strong inhibitor of mebendazole metabolism, based on human hepatocyte and human liver microsome *ex vivo* studies reported in the examples hereinbelow, where strong concentration-related inhibition was observed, in accordance with the definition of a strong CYP1A2 inhibitor as defined above.

Thiabendazole is also a preferred CYP1A2 inhibitor in the combinations of the present invention.

Thiabendazole has now been shown to be a strong inhibitor of mebendazole metabolism, based on studies reported in the examples hereinbelow, in accordance with the definition of a strong CYP1A2 inhibitor as defined above. Furthermore like mebendazole, thiabendazole is a benzimidazole drug with anti-proliferative including anti-cancer activity and anti-parasitic activity. Therefore, potential pharmacodynamic (anti-tumour and/or anti-proliferative efficacy) as well as pharmacokinetic (CYP1A2 inhibition of mebendazole metabolism) synergy may be observed with the mebendazole-thiabendazole combination of the present invention.

Fluvoxamine is also a preferred CYP1A2 inhibitor in the combinations of the present invention.

Fluvoxamine has now been shown to be a strong inhibitor of mebendazole metabolism, based on studies reported in the examples hereinbelow, in accordance with the definition of a strong CYP1A2 inhibitor as defined above. Furthermore, fluvoxamine has shown anti-tumour activity against glioblastoma by disrupting actin polymerization leading to suppression of glioblastoma migration and invasion (Hayashi et al., Scientific Reports, 2016, 6, 23372). As discussed already for mebendazole, the inhibition of tubulin polymerisation by mebendazole has been confirmed *in vitro* in a glioblastoma model. Therefore, potential pharmacodynamic (anti-tumour and/or anti-proliferative efficacy) as well as pharmacokinetic (CYP1A2 inhibition of mebendazole metabolism) synergy may be observed with the mebendazole-fluvoxamine combination of the present invention.

Strong or moderate CYP1A2 inhibitors are commercially available, or can be prepared by known methods or by analogy with known methods. For example, furafylline, thiabendazole and fluvoxamine are available from commercial suppliers including Sigma-Aldrich^{®}.

### Methods of treatment

Methods of treatment by therapy of a human subject are discussed in more detail below. As used herein the term "method of treatment" or "methods of treatment" refers to the treatment by therapy of a human subject of each of the aspects of the present invention. The following description is applicable to the methods of treatment of each of the aspects of the present invention.

By combining mebendazole with a strong or moderate CYP1A2 inhibitor, the combinations of the invention may improve the systemic bioavailability of mebendazole. The combinations of the invention may also improve the predictability of the systemic bioavailability and hence efficacy / therapeutic activity of mebendazole by reducing variability in systemic bioavailability of mebendazole between individual subjects and on an intra-subject basis.

Thus, mebendazole may be better absorbed from the gastrointestinal tract after oral dosing than had previously been thought, and the low systemic availability of mebendazole, as seen in humans, may be due to metabolism (in particular by CYP1A2 metabolism during first pass hepatic metabolism) to a much greater extent than previously thought. Systemic bioavailability of mebendazole may therefore be improved by administration with a strong or moderate CYP1A2 inhibitor. Inhibition of the metabolism of mebendazole by use of a moderate or strong CYP1A2 inhibitor, and not by use of a weak CYP1A2 inhibitor would be expected to reduce intra-subject and inter-subject (patient) variability in systemic therapeutic concentrations of mebendazole (as noted, for example, by Dayan, Acta Tropica., 2003, 86, 141). Intra-subject variation can occur by exposure to dietary and environmental compounds including for example cigarette smoke and oral contraceptive steroids which induce CYP1A2 (Zhou et al., 2009).

The combinations of the invention can be used to enable mebendazole to achieve the desired mechanisms of action systemically. Accordingly, in some embodiments, the method of treatment may comprise, systemically, one or more mechanism including but not limited to the following:
- microtubule inhibition
- inhibition of cell proliferation
- inhibition of angiogenesis
- inhibition of TRAF2- and NCK-interacting kinase (TNIK)
- interference with vascular epidermal growth factor receptor 2 (VEGFR2)
- induction of apoptosis
- inhibition of metalloproteinases
- activity at BCR-ABL kinase
- activity at BRAF kinase
- Hedgehog signalling pathway inhibition
- induction of proinflammatory (M1) phenotype of monocytoid cells
- antifungal activity, including anticryptococcal activity

In some embodiments, the method of treatment may be a method of treating a disease which is selected from haematological cancers, solid tumours including cancer and non-cancer disorders, inflammatory diseases, fibrotic diseases, gout, liver cirrhosis, scleroderma, psoriasis, endometriosis, and fungal diseases, e.g. Cryptococcus neoformans.

In some embodiments, the cancer or non-cancer tumor disorder may be selected from Bronchial Tumors, Central Nervous System Cancer, Central Nervous System Embryonal Tumors, Carcinoma, Acute Myeloid Leukemia (AML), Carcinoid Tumor, Appendix Cancer, Astrocytomas, Chordoma, Atypical Teratoid/Rhabdoid Tumor, Sarcoma, Bladder Cancer, Thyroid Cancer, Primary Central Nervous System (CNS) Lymphoma, Extracranial Germ Cell Tumor, Esophageal Cancer, AIDS-Related Cancers, Hepatocellular (Liver) Cancer, Penile Cancer, Pleuropulmonary Blastoma, Gallbladder Cancer, Rhabdomyosarcoma, Waldenström Macroglobulinemia, Salivary Gland Cancer, Central Nervous System Germ Cell Tumors, Plasma Cell Neoplasm, Lip and Oral Cavity Cancer, Testicular Cancer, Extrahepatic Bile Duct Cancer, Ductal Carcinoma In Situ (DCIS), Nasopharyngeal Cancer, Nasal Cavity and Paranasal Sinus Cancer, Bone Cancer, Breast Cancer, Glioma, Hairy Cell Leukemia, Langerhans Cell Histiocytosis, Oral Cancer, Ependymoma, Cutaneous T-Cell Lymphoma, Gestational Trophoblastic Disease, Eye Cancer, Kaposi Sarcoma, Extragonadal Germ Cell Tumor, Gastric (Stomach) Cancer, Gastrointestinal Stromal Tumors (GIST), Papillomatosis, Small Intestine Cancer, Brain and Spinal Cord Tumors, Waldenström Macroglobulinemia, Pancreatic Cancer, Pharyngeal Cancer, Oropharyngeal Cancer, Paraganglioma, Nonmelanoma Skin Cancer, Myelodysplastic/Myeloproliferative Neoplasms, Squamous Cell Carcinoma, Malignant Fibrous Histiocytoma, Melanoma, Sezary Syndrome, Merkel Cell Carcinoma, Pituitary Tumor, Malignant Fibrous Histiocytoma of Bone and Osteosarcoma, Ovarian Cancer, Parathyroid Cancer, Skin Cancer, Mycosis Fungoides, Germ Cell Tumor, Fallopian Tube Cancer, Intraocular Melanoma, Leukemia, Pancreatic Neuroendocrine Tumors (Islet Cell Tumors), Endometrial Cancer, Lymphoma, Prostate Cancer, Renal Pelvis and Ureter Cancer, Osteosarcoma (Bone Cancer), Non-Hodgkin Lymphoma, Non-Small Cell Lung Cancer, Basal Cell Carcinoma, Laryngeal Cancer, Multiple Myeloma/Plasma Cell Neoplasm, Vaginal Cancer, Squamous Neck Cancer, Multiple Myeloma, Midline Tract Carcinoma Involving NUT Gene, Head and Neck Cancer, Heart Cancer, Intraocular (Eye), Renal Cell (Kidney) Cancer, Malignant Fibrous Histiocytoma of Bone, Liver Cancer, Rectal Cancer, Colon Cancer, Malignant Mesothelioma, Low Malignant Potential Tumor, Mouth Cancer, Soft Tissue Sarcoma, Hypopharyngeal Cancer, Wilms Tumor, Epithelial Cancer, Ewing Sarcoma Family of Tumors, Acute Lymphoblastic Leukemia (ALL), Retinoblastoma, Hodgkin Lymphoma, Brain Tumor, Esthesioneuroblastoma, Embryonal Tumors, Cervical Cancer, Chronic Myeloproliferative Neoplasms, Pancreatic Neuroendocrine Tumors, Ureter and Renal Pelvis Cancer, Anal Cancer, Urethral Cancer, Brain Stem Cancer, Vulvar Cancer, Chronic Lymphocytic Leukemia (CLL), Uterine Sarcoma, Stomach (Gastric) Cancer, Brain Stem Glioma, Multiple Endocrine Neoplasia Syndromes, Myelodysplastic Syndromes, Craniopharyngioma, Small Cell Lung Cancer, Lip and Oral Cavity Cancer, Cutaneous T-Cell Lymphoma, Neuroblastoma, Acute Lymphoblastic Leukemia (ALL), Langerhans Cell Histiocytosis, Breast Cancer, Gastrointestinal Carcinoid Tumor, Paranasal Sinus and Nasal Cavity Cancer, Pheochromocytoma, Metastatic Squamous Neck Cancer with Occult Primary, Male Breast Cancer, Kidney (Renal) Cancer, Lung Cancer, Islet Cell Tumors, Extrahepatic Bile Duct Cancer, Endometrial Uterine Cancer, Chronic Myeloproliferative Neoplasms, Transitional Cell Cancer of the Renal Pelvis and Ureter, Thymoma and Thymic Carcinoma, Throat Cancer, Ewing Sarcoma, Chronic Myelogenous Leukemia (CML), Colorectal Cancer, Colon Cancer, Cardiac (Heart) Tumors, Burkitt Lymphoma, Carcinoma of Unknown Primary, Adrenocortical Carcinoma, Adrenocortical Adenocarcinoma, Adrenocortical Adenoma, Central Nervous System Atypical Teratoid/Rhabdoid Tumor, Childhood Cancers, Non-Hodgkin Lymphoma, and P-gp expressing Multidrug Resistant Tumors. In some embodiments, the cancer may be Sarcoma, Ovarian Cancer, Kidney (Renal) Cancer, Melanoma, Colorectal Cancer, Colon Cancer, Adrenocortical Carcinoma, Adrenocortical Adenocarcinoma, Adrenocortical Adenoma, P-gp expressing Multidrug Resistant Tumors, Neuroblastoma, non-cancer disorders, inflammatory diseases, fibrotic diseases, gout, liver cirrhosis, scleroderma, psoriasis, endometriosis, and fungal diseases e.g. Cryptococcus neoformans.

The subject of the methods of treatment is a human. The human may be an adult human. The human may be a child in which the CYP1A2 isoenzyme is active, even if it has not yet reached adult levels of activity. The World Health Organisation (WHO/CDS/CPE/PVC/2002.4, 2002) states that the metabolism and catabolism of mebendazole attains adult levels of activity in children aged from 10 to 24 months. Accordingly, in some embodiments the human subject maybe a child aged 10 months or older (e.g. 24 months or older). In some embodiments, the human subject maybe a child younger than 10 months with evidence of CYP1A2 activity.

The combinations of the present invention can be administered in a variety of dosage forms, for example orally such as in the form of tablets, capsules, sugar- or film-coated tablets, liquid solutions or suspensions or parenterally, for example intramuscularly, intravenously or subcutaneously. The combinations may therefore be given by injection, infusion, or by inhalation or nebulisation. The combinations are preferably given by oral administration.

The combinations of the present invention are preferably for (e.g. suitable for) oral administration.

The dosage of each active ingredient in the combinations of the present invention depends on a variety of factors including the age, weight and condition of the patient and the route of administration. Daily dosages can vary within wide limits and will be adjusted to the individual requirements in each particular case. Typically, however, the dosage adopted for each route of administration when a compound is administered alone to adult humans is 0.0001 to 650 mg/kg or 0.001 to 650 mg/kg, most commonly in the range of 0.001 to 75 mg/kg, 0.01 to 75 mg/kg or 0.001 to 10 mg/kg body weight. For example, the dosage adopted for each route of administration when a compound is administered alone to adult humans may be 0.05 to 25 mg/kg or 0.01 to 1 mg/kg. Such a dosage may be given, for example, from 1 to 5 times daily. For intravenous injection, a suitable daily dose is from 0.0001 to 50 mg/kg body weight, 0.0001 to 10 mg/kg body weight or 0.0001 to 1 mg/kg body weight. Preferably, for intravenous injection, the suitable daily dose is from 0.01 to 50 mg/kg body weight or preferably from 0.05 to 25 mg/kg body weight. A daily dosage can be administered as a single dosage or according to a divided dose schedule. A unit dose form such as a tablet or a capsule will usually contain 1-500 mg or 1-250 mg of active ingredient. For example, either of the active ingredients of the combinations of the present invention could be administered to a human patient at a dose of between 1-1500 mg, 1-1000 mg, 1-500 mg or 100-250 mg either once a day, twice or three times a day.

The dosage of the strong or moderate CYP1A2 inhibitor in the combinations of the invention is typically the lowest dosage required in order to achieve satisfactory inhibition of metabolism of mebendazole. The dosage of the strong or moderate CYP1A2 inhibitor in the combinations of the invention is typically lower than would be used if the CYP1A2 inhibitor were being administered as a single active ingredient for a therapeutic target(s) other than CYP1A2 inhibition. The ability to dose the strong or moderate CYP1A2 inhibitor at levels typically lower than if the strong or moderate CYP1A2 inhibitor were being administered as a single active ingredient may provide an advantageous reduction of side effects resulting from the strong or moderate CYP1A2 inhibitor. However, in certain circumstances, the CYP1A2 inhibitor (e.g. fluvoxamine or thiabendazole) may have therapeutic activity (e.g. anticancer activity) requiring a higher dosage to achieve additional therapeutic activity, over and above that required to achieve satisfactory CYP1A2 inhibition of mebendazole metabolism. Under such circumstances, the dosage of the CYP1A2 inhibitor may be increased to therapeutic levels above that required only for CYP1A2 inhibition.

The pharmaceutical composition of the invention may further comprise a pharmaceutically acceptable adjuvant, diluent or carrier. Conventional procedures for the selection and preparation of suitable pharmaceutical formulations are described in, for example, Pharmaceuticals - The Science of Dosage Form Designs, M. E. Aulton, Churchill Livingstone, 1988.

Depending on the mode of administration, the pharmaceutical composition will preferably comprise from 0.05 to 99% w/w (percent by weight), more preferably from 0.05 to 80% w/w, still more preferably from 0.10 to 70% w/w, and even more preferably from 0.10 to 50% w/w, of the combined weight of the active ingredients, all percentages by weight being based on total composition. The disclosure further provides a process for the preparation of a pharmaceutical composition of the invention which comprises mixing the active ingredients as hereinbefore described with a pharmaceutically acceptable adjuvant, diluent or carrier (not part of the claimed invention).

The combinations of the invention may be administered in a variety of dosage forms. Thus, they can be administered orally, for example as tablets, troches, lozenges, aqueous or oily suspensions, solutions, dispersible powders or granules. The combinations of the invention may also be administered parenterally, whether subcutaneously, intravenously, intramuscularly, intrasternally, transdermally, by infusion techniques or by inhalation or nebulisation. The combinations may also be administered as suppositories. Solid oral forms of the pharmaceutical composition of the invention may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulfates; and, in general, non-toxic and pharmacologically inactive substances used in pharmaceutical formulations. Such pharmaceutical preparations may be manufactured in known manner, for example, by means of mixing, granulating, tableting, sugar coating, or film coating processes. Liquid dispersions for oral administration may be solutions, syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, propylene glycol or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride. Further suitable carriers for suspensions include sterile water, hydroxypropyl-β-cyclodextrin, hydroxypropylmethyl cellulose (HPMC), polysorbate 80, polyvinylpyrrolidone (PVP), aerosol AOT (i.e. sodium 1,2-bis(2-ethylhexoxycarbonyl)ethanesulphonate), D-α-Tocopherol polyethylene glycol 1000 succinate, pluronic F127 and/or captisol (i.e. sulfobutylether-beta-cyclodextrin).

The compounds of the invention may, for example, be formulated as aqueous suspensions or aqueous solutions in a carrier containing one or more of the following excipients at concentrations such as:
(i) 0.5% w/v hydroxypropylmethyl cellulose (HPMC);
(ii) 0.67% w/v polyvinylpyrrolidone (PVP)/0.33% w/v aerosol AOT (sodium 1,2-bis(2-ethylhexoxycarbonyl)ethanesulphonate);
(iii) 1 % w/v pluronic F 127;
(iv) 30% w/v propylene glycol;
(v) 40% w/v hydroxypropyl-β-cyclodextrin;
(vi) 40% w/v β-cyclodextrin;
(vii) 40% w/v methyl-β-cyclodextrin;
viii) 40% w/v captisol (i.e. sulfobutylether-β-cyclodextrin).
(ix) 0.5% w/v polysorbate 80; and
(x) 10% w/v polyethylene glycol (PEG)
(xi) 20% w/v D-α-Tocopherol polyethylene glycol 1000 succinate

The carriers may be prepared by standard procedures known to those of skill in the art. For example, each of the carriers (i) to (xi) may be prepared by weighing the required amount of excipient into a suitable vessel, adding approximately 80% of the final volume of water and magnetically or mechanically stirring until a solution is formed. The carrier is then made up to volume with water. The aqueous suspensions of the active ingredients of the combination may be prepared by weighing the required amount of each active ingredient into a suitable vessel, adding 100% of the required volume of carrier and magnetically or mechanically stirring. Solutions for injection or infusion may contain as carrier, for example, sterile water or preferably they may be in the form of sterile, aqueous, isotonic saline solutions.

### Certain embodiments of the invention

Certain embodiments of the invention are described in more detail below. Unless otherwise specified, the embodiments described below are applicable to each of the aspects of the present invention.

The pharmaceutical composition comprises mebendazole or a pharmaceutically acceptable salt, solvate, hydrate, or N-oxide thereof and a strong or moderate CYP1A2 inhibitor.

In some embodiments:
- the strong or moderate CYP1A2 inhibitor is furafylline, thiabendazole, fluvoxamine, 8-phenyltheophylline, ciprofloxacin, enoxacin, or zafirlukast, or a pharmaceutically acceptable salt, solvate, hydrate or N-oxide thereof.

In some embodiments:
- mebendazole has an IC50 of 1 µM or less against a neuroblastoma, sarcoma, renal cancer or ovarian cancer-derived cell line in a cell viability assay; and
- the strong or moderate CYP1A2 inhibitor is furafylline or a pharmaceutically acceptable salt, solvate, hydrate or N-oxide thereof.

In some embodiments:
- the strong or moderate CYP1A2 inhibitor is furafylline or a pharmaceutically acceptable salt, solvate, hydrate or N-oxide thereof.

In some embodiments:
- the strong or moderate CYP1A2 inhibitor is thiabendazole or a pharmaceutically acceptable salt, solvate, hydrate or N-oxide thereof.

In some embodiments:
- the strong or moderate CYP1A2 inhibitor is fluvoxamine or a pharmaceutically acceptable salt, solvate, hydrate or N-oxide thereof.

In some embodiments:
- the strong or moderate CYP1A2 inhibitor is furafylline.

In some embodiments:
- the strong or moderate CYP1A2 inhibitor is thiabendazole.

In some embodiments:
- the strong or moderate CYP1A2 inhibitor is fluvoxamine.

In some embodiments of the aspects of the present invention:
- the strong or moderate CYP1A2 inhibitor is furafylline, thiabendazole, 8-phenyltheophylline, ciprofloxacin, enoxacin, fluvoxamine, or zafirlukast, or a pharmaceutically acceptable salt, solvate, hydrate or N-oxide thereof; and
- the method of treatment is a method of treatment of a disease selected from sarcoma, ovarian cancer, kidney (renal) cancer, melanoma, colon cancer, lung cancer, brain cancer, adrenocortical carcinoma, adrenocortical adenocarcinoma, adrenocortical adenoma, a P-gp expressing multidrug resistant tumour, neuroblastoma, a solid tumour disorder (including cancer and non-cancer disorders), a haematological cancer, an inflammatory disease, a fibrotic disease, gout, liver cirrhosis, scleroderma, psoriasis, endometriosis, and any fungal disease e.g. Cryptococcus neoformans.

In some embodiments of the aspects of the present invention:
- the method of treatment is a method of treatment of a disease selected from sarcoma, ovarian cancer, kidney (renal) cancer, melanoma, colorectal cancer, colon cancer, lung cancer, brain cancer, adrenocortical carcinoma, adrenocortical adenocarcinoma, adrenocortical adenoma, a P-gp expressing multidrug resistant tumour, neuroblastoma, a solid tumour disorder (including cancer and non-cancer disorders), a haematological cancer, an inflammatory disease, a fibrotic disease, gout, liver cirrhosis, scleroderma, psoriasis, endometriosis, and any fungal disease e.g. Cryptococcus neoformans.
- In some embodiments of the aspects of the present invention:
- the strong or moderate CYP1A2 inhibitor is furafylline, thiabendazole or fluvoxamine; and
- the method of treatment is a method of treatment of a disease selected from sarcoma, ovarian cancer, kidney (renal) cancer, melanoma, colorectal cancer, colon cancer, lung cancer, brain cancer, adrenocortical carcinoma, adrenocortical adenocarcinoma, adrenocortical adenoma, a P-gp expressing multidrug resistant tumour, neuroblastoma, a solid tumour disorder (including cancer and non-cancer disorders), a haematological cancer, an inflammatory disease, a fibrotic disease, gout, liver cirrhosis, scleroderma, psoriasis, and endometriosis.

In some embodiments of the aspects of the present invention:
- the strong or moderate CYP1A2 inhibitor is furafylline, fluvoxamine or thiabendazole; and
- the method of treatment is a method of treatment of a disease selected from sarcoma, ovarian cancer, kidney (renal) cancer, melanoma, colon cancer, lung cancer, brain cancer, adrenocortical carcinoma, adrenocortical adenocarcinoma, adrenocortical adenoma, a P-gp expressing multidrug resistant tumour and neuroblastoma.

In some embodiments of the aspects of the present invention:
- the strong or moderate CYP1A2 inhibitor is furafylline, thiabendazole or fluvoxamine; and
- the method of treatment is a method of treatment of a disease selected from a solid tumour disorder (including cancer and non-cancer disorders), a haematological cancer, an inflammatory disease, a fibrotic disease, gout, liver cirrhosis, scleroderma, psoriasis, and endometriosis.

In some embodiments of the aspects of the present invention:
- the strong or moderate CYP1A2 inhibitor is furafylline, thiabendazole or fluvoxamine; and
- the method of treatment is a method of treatment of a fungal disease e.g. Cryptococcus neoformans.

### Examples

### Example 1: Study to Investigate Whether Mebendazole is a Substrate of the Human Efflux Transporters P-gp and BCRP in Polarised Caco-2 Cell Monolayers

The objective of this study was to assess the potential of mebendazole to be a substrate of the human efflux transporters P-gp and BCRP in polarised Caco-2 cell monolayers. Test compound (at 10 µM in the absence and presence of a reference inhibitor) was incubated with the polarised cell monolayer for 120 min. Bidirectional apparent permeability of test compound was quantified by LC-MS/MS and used to determine an efflux ratio.

Efflux ratios of positive control substrates in the absence and presence of reference inhibitor were determined from incubations run alongside test compound and confirmed that the in vitro test system was capable of detecting transported substrates. For P-gp substrate assessment, the efflux ratio obtained for talinolol in the absence (ER = 52.2) and presence (ER = 1.48) of reference inhibitor confirmed that the in vitro test system was capable of detecting substrates of P-gp.

Calculated mean % recovery values for talinolol, atenolol and propranolol were acceptable. Mebendazole calculated mean % recovery values ranged from 55.7 to 75.1 %, indicating that there were no major issues of non-specific binding. Mebendazole calculated mean % recovery value A-B in the absence of inhibitor was 55.7 %, but A-B Papp values were consistent regardless of recovery across the study giving confidence in the interpretation of results.

The mean determined A-B and B-A Pₐₚₚ values (cm/s ×10⁻⁶) for mebendazole were 25.4 and 29.8, respectively, giving an efflux ratio of 1.17 (Table 1). In the presence of the P-gp reference inhibitor verapamil, the calculated efflux ratio was 1.16. These results confirmed that mebendazole is not a substrate of the human P-gp transporter.

**Table 1: Bidirectional apparent permeability of test compounds across Caco-2 cell monolayers in the absence and presence of the P gp reference inhibitor verapamil**

| **Compound (JIM)** | **Direction** | **Pₐₚₚ (x 10⁻⁶ cms⁻¹)** | | | **Mean % Recovery** | **Efflux Ratio** |
|---|---|---|---|---|---|---|
| | | **Rep 1** | **Rep 2** | **Mean** | | |
| Mebendazole (10 µM) | A-B | 22.4 | 28.5 | 25.4 | 55.7 | 1.17 |
| | B-A | 30.2 | 29.4 | 29.8 | 75.1 | |
| Mebendazole (10 µM) + Verapamil (100 µM) | A-B | 25.9 | 32.7 | 29.3 | 68.0 | 1.16 |
| | B-A | 34.4 | 33.9 | 34.1 | 79.3 | |

For BCRP substrate assessment, the efflux ratio obtained for estrone-3-sulfate in the absence (ER = 128) and presence (ER = 1.96) of reference inhibitor confirmed that the *in vitro* test system was capable of detecting substrates of BCRP.

Calculated mean % recovery values for estrone-3-sulfate, atenolol and propranolol were acceptable. Mebendazole calculated mean % recovery values ranged from 52.0 to 75.7 %, indicating that there were no major issues of non-specific binding. Mebendazole calculated mean % recovery value A-B in the absence of inhibitor was 52.0 %, but A-B Pₐₚₚ values were consistent regardless of recovery across the study giving confidence in the interpretation of results.

The mean determined A-B and B-A Pₐₚₚ values (cm/s ×10⁻⁶) for mebendazole were 23.5 and 29.0, respectively, giving an efflux ratio of 1.23 (Table 2). In the presence of the BCRP reference inhibitor fumitremorgin C, the calculated efflux ratio was 1.25. These results confirmed that mebendazole is not a substrate of the human BCRP transporter.

**Table 2: Bidirectional apparent permeability of test compounds across Caco-2 cell monolayers in the absence and presence of the BCRP reference inhibitor fumitremorgin C (FTC)**

| **Compound (µM)** | **Direction** | **Pₐₚₚ (x 10⁻⁶ cms⁻¹)** | | | **Mean % Recovery** | **Efflux Ratio** |
|---|---|---|---|---|---|---|
| | | **Rep 1** | **Rep 2** | **Mean** | | |
| | | | | | | |
| Mebendazole (10 µM) | A-B | 19.7 | 27.4 | 23.5 | 52.0 | 1.23 |
| | B-A | 28.2 | 29.8 | 29.0 | 75.7 | |
| Mebendazole (10 µM) + FTC (10 µM) | A-B | 23.0 | 30.6 | 26.8 | 64.5 | 1.25 |
| | B-A | 31.4 | 35.3 | 33.3 | 80.9 | |

Under the assay conditions with the in vitro cell test system utilised in this study, mebendazole has high passive permeability and has been determined not to be a substrate of either P-gp or BCRP.

These data suggest that, contrary to what has previously been thought in some cases, oral absorption of mebendazole may be higher than assumed. The reduced systemic bioavailability of mebendazole, as seen in humans, may be due to metabolism to a much greater extent than previously thought.

### Example 2: Study to Investigate Whether Mebendazole is a Substrate of the Human SLC Transporters OATP1B1 and OATP1B3 in Transiently Transfected HEK293 Cells

The objective of this study was to assess the potential of mebendazole to be a substrate of human SLC transporters (OATP1B1 and OATP1B3) in transfected HEK293 cells. Test compound (at 1 and 10 µM) was incubated with both SLC transporter-expressing HEK293 cells and control (empty vector) HEK293 cells for 2 min. Uptake of test compound was quantified by LC-MS/MS and used to determine an uptake ratio. Uptake ratios of positive control substrates were determined from incubations run alongside test compound and confirmed that the in vitro test systems were capable of detecting transported substrates.

The uptake ratios obtained for [3H]-estradiol 17β-D-glucuronide confirmed that the in *vitro* test system was capable of detecting substrates of OATP1B1. Calculated uptake ratios were 0.9 and 0.9 for 1 µM and 10 µM mebendazole, respectively, confirming that mebendazole is not a substrate of the human OATP1B1 transporter (Table 3).

The uptake ratios obtained for [3H]-estradiol 17β-D-glucuronide confirmed that the in *vitro* test system was capable of detecting substrates of OATP1B3. Calculated uptake ratios were 1.1 and 1.1 for 1 µM and 10 µM mebendazole, respectively, confirming that mebendazole is not a substrate of the human OATP1B3 transporter (Table 4).

**Table 3: Uptake rate of Mebendazole into OATP1B1 and empty vector (control) transfected HEK293 cells**

| **Test Compound** | **Concentration (µM)** | **Cell line** | **Uptake rate (pmol/mg)** | | **Uptake ratio** |
|---|---|---|---|---|---|
| | | | **Mean** | **SD** | |
| Mebendazole | 1 | OATP1B1 | 42.5 | 8.08 | 0.9 |
| | | Control | 49.0 | 8.11 | |
| | 10 | OATP1B1 | 426 | 52.3 | 0.9 |
| | | Control | 461 | 35.5 | |

**Table 4: Uptake rate of Mebendazole into OATP1B3 and empty vector (control) transfected HEK293 cells**

| **Test Compound** | **Concentration (µM)** | **Cell line** | **Uptake rate (pmol/mg)** | | **Uptake ratio** |
|---|---|---|---|---|---|
| | | | **Mean** | **SD** | |
| Mebendazole | 1 | OATP1B3 | 56.6 | 5.14 | 1.1 |
| | | Control | 50.1 | 6.40 | |
| | 10 | OATP1B3 | 466 | 30.7 | 1.1 |
| | | Control | 414 | 16.6 | |

Under the current assay conditions with the *in vitro* transfected cell test systems utilised in this study, mebendazole has been determined not to be a substrate of OATP1B1 or OATP1B3.

OATP1B1 is an uptake transporter exclusively expressed on the sinusoidal side of hepatocytes, and is responsible for the hepatic uptake of drugs. OATP1B3 is an uptake transporter exclusively expressed in the liver on the sinusoidal (basolateral) side of centrilobular hepatocytes. In conjunction with OATP1B1, it is responsible for the hepatic uptake of some drugs.

### Example 3: Study to Investigate the Cytochrome P450 Reaction Phenotyping of the Test Compound, Mebendazole, Using Recombinant Enzymes

The objective of this study was to determine the stability of the test compound, mebendazole, in the presence of CYP1A2, CYP2B6, CYP2C8, CYP2C9, CYP2C19, CYP2D6 and CYP3A4 recombinant cytochrome P₄₅₀ enzymes.

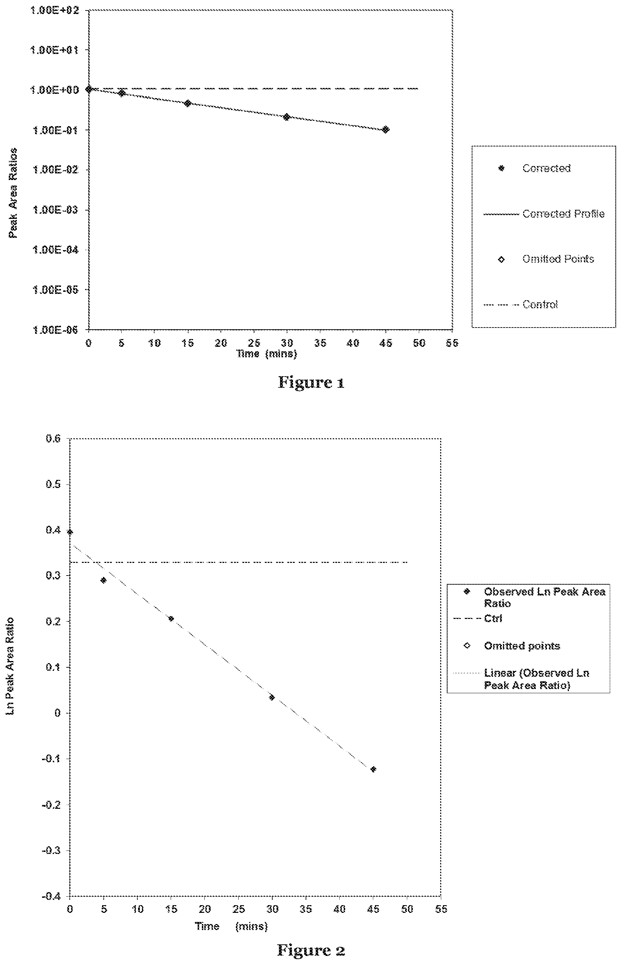

Bactosomes^{™} for each P450 isoform, 0.1 M phosphate buffer pH 7.4 and test compound (final substrate concentration 5 µM) were pre-incubated at 37 °C prior to the addition of NADPH (final concentration 1 mM) to initiate the reaction. Incubations were also performed using control Bactosomes^{™} (no P450 enzymes present) to reveal any non-enzymatic degradation. Control compounds known to be metabolised specifically by each P450 isoform were included. All incubations were performed singularly for each test compound. Each compound was incubated for 0, 5, 15, 30 and 45 min with each isoform, and analysed using LC-MS/MS. The control compounds behaved as expected in the assays. The results for mebendazole can be found in Table 5, and specifically for CYP1A2 in Figure 1.

Mebendazole gave half-life values of 13.3 min and 93.0 min in the presence of CYP1A2 and CYP2C19 recombinant enzymes, respectively. There was little or no turnover half-life (t_{1/2}) > 135 min, % compound remaining > 87.5 %) of mebendazole in the presence of CYP2B6, CYP2C8, CYP2C9, CYP2D6 or CYP3A4 recombinant enzymes.

**Table 5: Cytochrome P450 reaction phenotyping data for Mebendazole**

| **Compound** | **Isoform** | **Cytochrome P450 Reaction Phenotyping** | | | | | | | |
|---|---|---|---|---|---|---|---|---|---|
| | | **t_{½} (min)** | **SE t_{½} (min)** | **n** | **Compound Remaining (% of o min)** | | | | |
| | | | | | **0 min** | **5 min** | **15 min** | **30 min** | **45 min** |
| Mebendazole | 1A2 | 13.3 | 0.173 | 5 | 100 | 80.1 | 45.8 | 20.7 | 9.88 |
| | 2B6 | >135 | - | 5 | 100 | 117 | 108 | 112 | 113 |
| | 2C8 | >135 | - | 5 | 100 | 90.6 | 93.4 | 102 | 99.1 |
| | 2C9 | >135 | - | 5 | 100 | 108 | 108 | 109 | 105 |
| | 2C19 | 93.0 | 12.9 | 5 | 100 | 92.6 | 82.1 | 76.3 | 70.5 |
| | 2D6 | >135 | - | 5 | 100 | 93.6 | 93.3 | 94.6 | 90.4 |
| | 3A4 | >135 | - | 5 | 100 | 95.1 | 85.4 | 105 | 104 |

The stability of mebendazole in the presence of CYP1A2 recombinant P450 isoform is shown in Figure 1.

In conclusion, mebendazole was metabolised by recombinant enzymes for CYP1A2 and, to much a lesser extent, CYP2C19, but not CYP2B6, CYP2C8, CYP2C9, CYP2D6 or CYP3A4 (half-life values >135 min).

### Example 4: Study to Investigate the Stability of the Test Compound, Mebendazole, in Human Liver Microsomes in the Absence and Presence of the Inhibitors Cimetidine and Furafylline

The objective of this study was to determine and comparatively assess the stability of mebendazole in human liver microsomes in the absence of inhibitor and in the presence of the inhibitors cimetidine (30, 100 and 300 µM) or furafylline (3, 10 and 30 µM).

For metabolic stability investigations, human liver microsomes (final protein concentration 0.5 mg/mL), 0.1 M phosphate buffer pH 7.4 and mebendazole or phenacetin (final substrate concentration 1 µM) were pre-incubated at 37 °C prior to the addition of NADPH (final concentration 1 mM) to initiate the reaction. A minus cofactor control incubation was included for each compound tested where 0.1 M phosphate buffer pH 7.4 was added instead of NADPH (minus NADPH). Each test compound was incubated for 0, 5, 15, 30 and 45 min. The control (minus NADPH) was incubated for 45 min only. The samples were analysed for mebendazole or phenacetin using LC-MS/MS.

Mebendazole gave an intrinsic clearance (CLᵢₙₜ) value of 22.2 µL/min/mg protein in the absence of inhibitor in human liver microsomes.

Phenacetin, a CYP1A2 probe substrate, gave an intrinsic clearance value of 22.4 µL/min/mg protein in the absence of inhibitor in human liver microsomes.

For cytochrome P450 reaction phenotyping by chemical inhibitors (furafylline and cimetidine), human liver microsomes (final protein concentration 0.5 mg/mL), specific inhibitor (final methanol concentration 0.08 %), 0.1 M phosphate buffer pH 7.4 and mebendazole or phenacetin (final substrate concentration 1 µM) were pre-incubated at 37 °C prior to the addition of NADPH (final concentration 1 mM) to initiate the reaction. Test compound (mebendazole or the CYP1A2 substrate, phenacetin) was incubated without inhibitor and in the presence of each specific inhibitor concentration. A minus cofactor control incubation was included for each compound tested where 0.1 M phosphate buffer pH 7.4 was added instead of cofactor (minus NADPH). The specific inhibitors were cimetidine (pan P₄₅₀ inhibitor, final concentration 30, 100 and 300 µM) and furafylline (time dependent inhibitor of CYP1A2, final concentration 3, 10 and 30 µM). Each test compound was incubated for 0, 5, 15, 30 and 45 min. The control (minus NADPH) was incubated for 45 min only. The samples were analysed for mebendazole or phenacetin using LC-MS/MS.

Mebendazole clearance showed a slight, yet dose dependent decrease in the presence of cimetidine with intrinsic clearance values of 18.0, 17.8 and 15.0 µL/min/mg protein in the presence of 30, 100 and 300 µM cimetidine respectively.

Phenacetin clearance showed a slight, yet dose dependent decrease in the presence of cimetidine with intrinsic clearance values of 21.2, 17.6 and 13.9 µL/min/mg protein in the presence of 30, 100 and 300 µM cimetidine respectively.

Mebendazole clearance showed a marked dose dependent decrease in the presence of furafylline with intrinsic clearance values of 2.84, 0.232 and 0.336 µL/min/mg protein in the presence of 3, 10 and 30 µM furafylline respectively.

Phenacetin clearance showed a dose dependent decrease in the presence of furafylline with intrinsic clearance values of 3.49, 2.58 and 2.71 µL/min/mg protein in the presence of 3, 10 and 30 µM furafylline respectively.

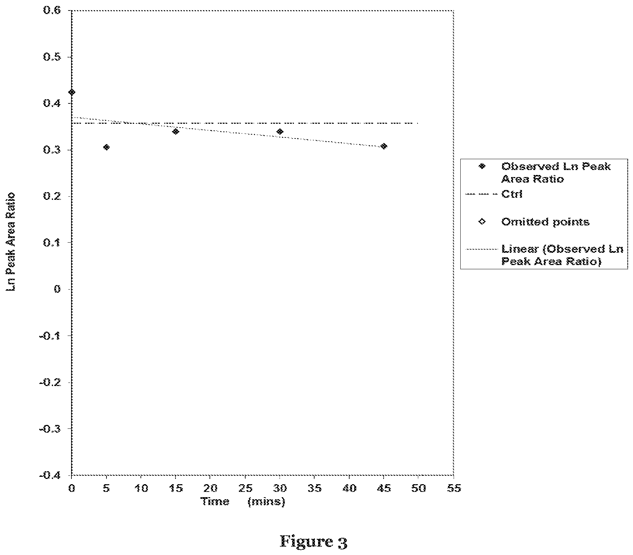

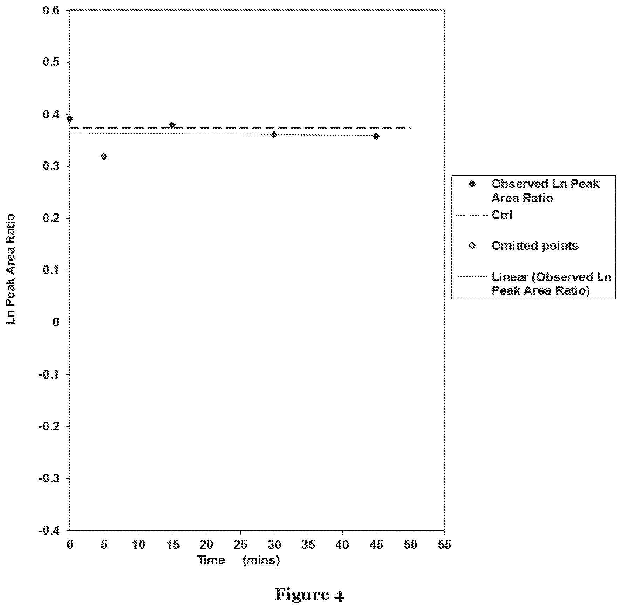

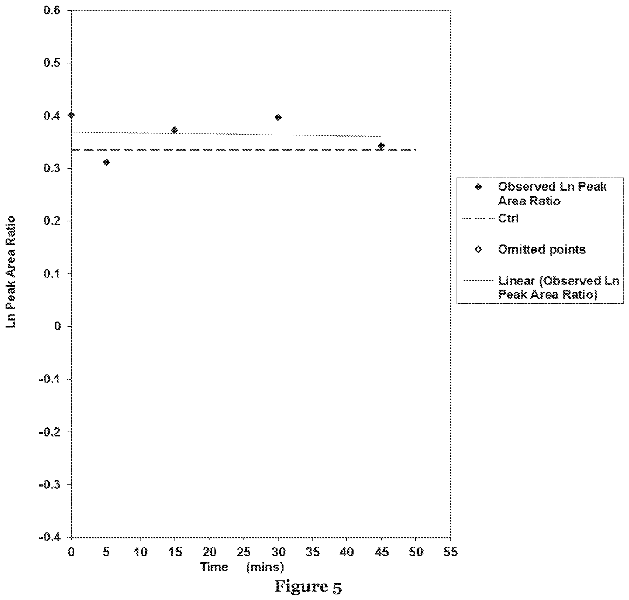

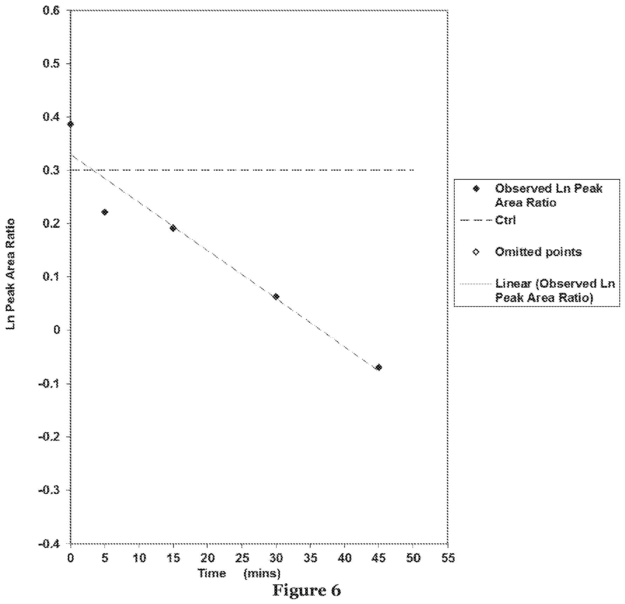

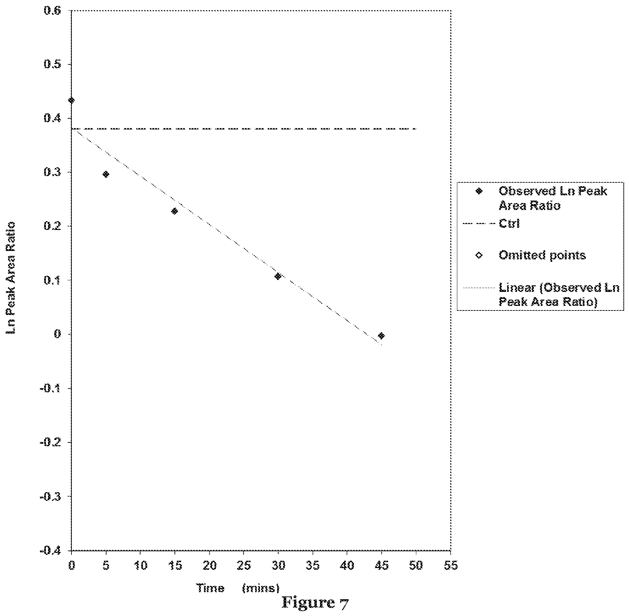

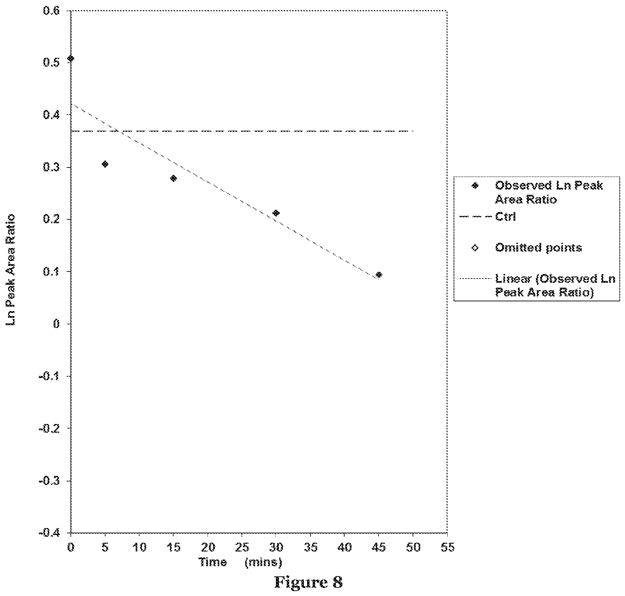

The results for this study are summarized in Table 6 and in Figures 2 to 8.

**Table 6: Reaction phenotyping (with chemical inhibitors, cimetidine and furafylline) data for mebendazole, and the control compound, phenacetin**

| **Compound** | **Inhibitor Cone (µM)** | **CYP450 Reaction Phenotyping (Chemical Inhibitors) (Species=Human)** | | | | |
|---|---|---|---|---|---|---|
| | | **CLᵢₙₜ (µL/min/mg protein)** | **SE CLᵢₙₜ** | **t_{½} (min)** | **n** | **Comments** |
| Mebendazole | None | 22.2 | 1.12 | 62.5 | 5 | No Inhibitor. |
| | Cimetidine 30 µM | 18.0 | 2.66 | 77.0 | 5 | Plus 30 µM cimetidine. |
| | Cimetidine 100 µM | 17.8 | 2.23 | 77.8 | 5 | Plus too µM cimetidine. |
| | Cimetidine 300 µM | 15.0 | 3.81 | 92.5 | 5 | Plus 300 µM cimetidine. |
| | Furafylline 3 µM | 2.84 | 2.50 | 488 | 5 | Plus 3 µM furafylline. |
| | Furafylline 10 µM | 0.232 | 1.70 | 5980 | 5 | Plus 10 µM furafylline. |
| | Furafylline 30 µM | 0.336 | 2.37 | 4120 | 5 | Plus 30 µM furafylline. |
| Phenacetin | None | 22.4 | 2.11 | 61.9 | 5 | No inhibitor. |
| | Cimetidine 30 µM | 21.2 | 1.24 | 65.3 | 5 | Plus 30 µM cimetidine. |
| | Cimetidine 100 µM | 17.6 | 1.45 | 78.9 | 5 | Plus too µM cimetidine. |
| | Cimetidine 300 µM | 13.9 | 2.30 | 99.6 | 5 | Plus 300 µM cimetidine. |
| | Furafylline 3 µM | 3.49 | 1.38 | 397 | 5 | Plus 3 µM furafylline. |
| | Furafylline 10 µM | 2.58 | 1.99 | 537 | 5 | Plus 10 µM furafylline. |
| | Furafylline 30 µM | 2.71 | 1.45 | 511 | 5 | Plus 30 µM furafylline. |

In conclusion, mebendazole clearance in human liver microsomes was decreased in the presence of the strong CYP1A2 inhibitor furafylline (96 fold (99% inhibition of intrinsic clearance)). Mebendazole clearance in human liver microsomes was decreased to a much lesser extent in the presence of cimetidine (reference compound) (1.5 fold (32% inhibition of intrinsic clearance)).

### Example 5: Study to Investigate the Stability of the Test Compound, Mebendazole, in Human Cryopreserved Hepatocytes in the Absence and Presence of the Inhibitors Cimetidine and Furafylline

The objective of this study was to investigate the stability of the test compound, mebendazole, in the presence of human cryopreserved hepatocytes in the absence of inhibitor and in the presence of cimetidine (30, 100 or 300 µM) or furafylline (3, 10 or 30 µM).

For hepatocyte stability investigations, Williams E media supplemented with 2 mM L-glutamine and 25 mM HEPES and test compound (final substrate concentration 1 µM; final DMSO concentration 0.25 %) were pre incubated at 37 °C prior to the addition of a suspension of cryopreserved hepatocytes (final cell density 0.5 × 10⁶ viable cells/mL in Williams E media supplemented with 2 mM L glutamine and 25 mM HEPES) to initiate the reaction. The final incubation volume was 500 µL. Two control compounds were included with each species, alongside appropriate vehicle control.

The reactions were stopped by transferring 50 µL of incubate to 100 µL acetonitrile containing internal standard at the appropriate time points (0, 5, 10, 20, 40 and 60 min). The termination plates were centrifuged at 2500 rpm at 4 °C for 30 min to precipitate the protein. The samples were analysed for mebendazole or ethoxycoumarin using LC-MS/MS.

Mebendazole gave an intrinsic clearance value of 19.1 uL/min/10⁶ cells in the presence of human cryopreserved hepatocytes.

For hepatocyte stability investigations in the presence of inhibitors, Williams E media supplemented with 2 mM L-glutamine and 25 mM HEPES, test compound (final substrate concentration 1 µM; final DMSO concentration 0.25 %) and selected CYP inhibitor (final methanol concentration 0.08 %) were pre incubated at 37 °C prior to the addition of a suspension of cryopreserved hepatocytes (final cell density 0.5 × 10⁶ viable cells/mL in Williams E media supplemented with 2 mM L glutamine and 25 mM HEPES) to initiate the reaction. The final incubation volume was 500 µL. Test compound was incubated without inhibitor and in the presence of cimetidine (pan P₄₅₀ inhibitor, final concentration 30, 100 and 300 µM) and furafylline (specific inhibitor of CYP1A2, final concentration 3, 10 and 30 µM).

A positive control compound, ethoxycoumarin, for CYP1A2 was included. The positive control compound was incubated without inhibitor and in the presence of each requested inhibitor. The reactions were stopped by transferring 50 µL of incubate to 100 µL acetonitrile containing internal standard at the appropriate time points (0, 5, 10, 20, 40 and 60 min). The termination plates were centrifuged at 2500 rpm at 4 °C for 30 min to precipitate the protein. The samples were analysed for mebendazole or ethoxycoumarin using LC-MS/MS.

Two control compounds were included in the assay and if the values for these compounds were not within the specified limits, the results were rejected and the experiment repeated.

Mebendazole gave an intrinsic clearance value of 13.3 µL/min/10⁶ cells in the presence of human cryopreserved hepatocytes in the absence of inhibitor.

Mebendazole gave intrinsic clearance values of 13.2, 14.7 and 9.98 µL/min/10⁶ cells in the presence of human cryopreserved hepatocytes with 30, 100 and 300 µM cimetidine respectively.

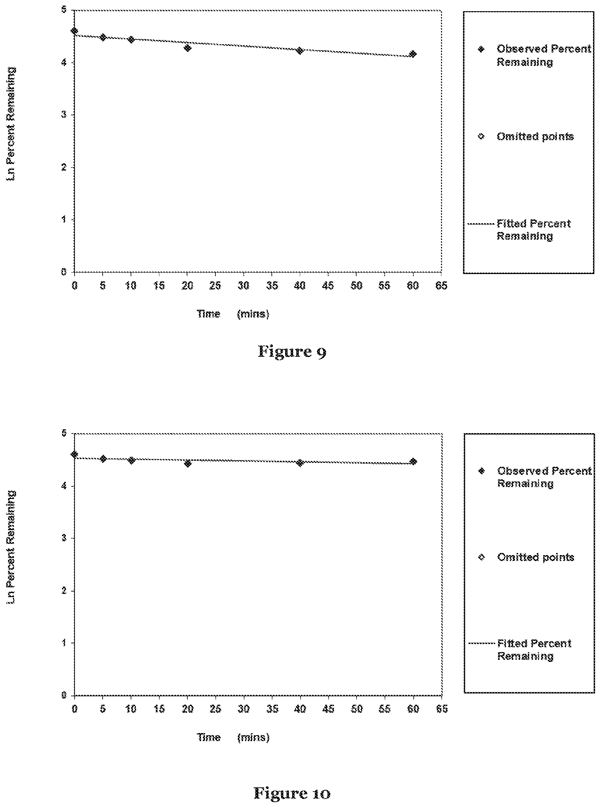

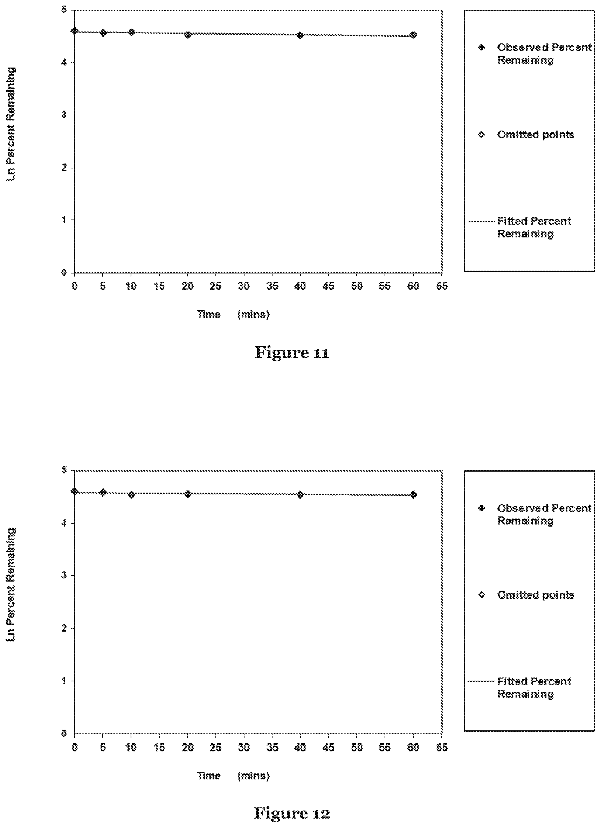

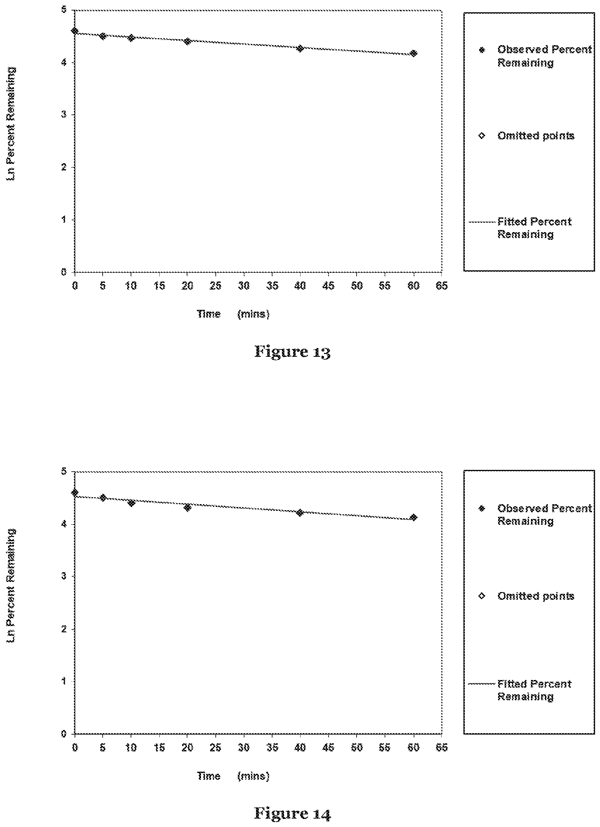

Mebendazole gave intrinsic clearance values of 3.47, 2.43 and 1.47 µL/min/10⁶ cells in the presence of human cryopreserved hepatocytes with 3, 10 and 30 µM furafylline respectively. The results are shown in Table 7 and Figures 9 to 15.

**Table 7: Hepatocyte stability (in the presence of chemical inhibitors) data for mebendazole and for the control compounds**

| **Compound** | **Inhibitor Cone (µM)** | **Hepatocyte Stability (Species=Human)** | | | | |
|---|---|---|---|---|---|---|
| | | **CLᵢₙₜ (µL/min/mg protein)** | **SE CLᵢₙₜ** | **t_{½} (min)** | **n** | **Comments** |
| Mebendazole | None | 13.3 | 2.88 | 104 | 6 | |
| | Cimetidine 30 µM | 13.2 | 1.29 | 105 | 6 | |
| | Cimetidine 100 µM | 14.7 | 2.27 | 94.1 | 6 | |
| | Cimetidine 300 µM | 9.98 | 1.37 | 139 | 6 | |
| | Furafylline 3 µM | 3.47 | 2.09 | 399 | 6 | |
| | Furafylline 10 µM | 2.43 | 0.873 | 570 | 6 | |
| | Furafylline 30 µM | 1.47 | 0.854 | 941 | 6 | |
| Verapamil | None | 85.7 | 4.32 | 16.2 | 4 | 40 and 60 minute time points excluded. |
| Umbelliferone | None | 174 | 6.90 | 7.95 | 4 | 40 and 60 minute time points excluded. |
| Phenacetin | None | 108 | 5.28 | 12.9 | 5 | 60 minute time point excluded. |
| | Cimetidine 30 µM | 104 | 5.10 | 13.4 | 6 | |
| | Cimetidine 100 µM | 88.5 | 3.65 | 15.7 | 6 | |
| | Cimetidine 300 µM | 73.8 | 3.21 | 18.8 | 6 | |
| | Furafylline 3 µM | 23.3 | 3.03 | 59.6 | 6 | |
| | Furafylline 10 µM | 15.5 | 2.52 | 89.3 | 6 | |
| | Furafylline 30 µM | 12.6 | 1.84 | 110 | 6 | |

In conclusion, metabolism of mebendazole by cryopreserved human hepatocytes was markedly decreased in the presence of the CYP1A2 inhibitor furafylline (9.1 fold (89% inhibition of intrinsic clearance)) and to a much lesser extent in the presence of cimetidine (reference compound) (1.3 fold (25% of inhibition of intrinsic clearance)). Half-lives of mebendazole were up to 139 minutes in the presence of cimetidine and up to 941 minutes in the presence of furafylline, compared to 104 minutes without any inhibitor.

### Example 6: Study to Investigate the Stability of the Test Compound, Mebendazole, in Human Cryopreserved Hepatocytes in the Absence and Presence of the Inhibitors Cimetidine and Thiabendazole

The objective of this study was to determine the stability of the test compound, mebendazole, in the presence of human cryopreserved hepatocytes in the absence and presence of cimetidine (30, 100 and 300 µM) and thiabendazole (10, 30 and 100 µM).

Mebendazole (1 µM) was incubated with cryopreserved human hepatocytes for 60 min in the absence and presence of cimetidine at 30, 100 and 300 µM) and thiabendazole at 10, 30 and 100 µM and samples taken at the required time points. The amount of test compound remaining at each time point was analysed by LC-MS/MS. Appropriate control compounds were also incubated along with ethoxycoumarin, which was incubated in the absence and presence of cimetidine and thiabendazole.

In the hepatocyte stability investigations, Williams E media supplemented with 2 mM L-glutamine and 25 mM HEPES, test compound (final substrate concentration 1 µM; final DMSO concentration 0.25 %) and, where applicable, the selected inhibitor (cimetidine at 30, 100 or 300 µM or thiabendazole at 10, 30 or 100 µM, final methanol concentration 0.08 %), were pre incubated at 37 °C prior to the addition of a suspension of cryopreserved hepatocytes (final cell density 0.5 × 10⁶ viable cells/mL in Williams E media supplemented with 2 mM L glutamine and 25 mM HEPES) to initiate the reaction. The final incubation volume was 500 µL. Two control compounds were included, alongside appropriate vehicle control. A positive control compound, ethoxycoumarin, for CYP1A2 was included. The positive control compound was incubated without inhibitor and in the presence of each inhibitor. The reactions were stopped by transferring 50 µL of incubate to 100 µL acetonitrile containing internal standard at the appropriate time points (0, 5, 10, 20, 40 and 60 min). The termination plates were centrifuged at 2500 rpm at 4 °C for 30 min to precipitate the protein.

Two control compounds were included in the assay and if the values for these compounds were not within the specified limits (where available) the results were rejected and the experiment repeated.

The positive control compounds behaved as expected in the assay and all intrinsic clearance values were within acceptable limits where applicable.

Mebendazole was metabolised by cryopreserved hepatocytes from human. Mebendazole gave an intrinsic clearance value of 15.0±0.658 µL/min/10⁶ cells in the absence of inhibitor in human cryopreserved hepatocytes.

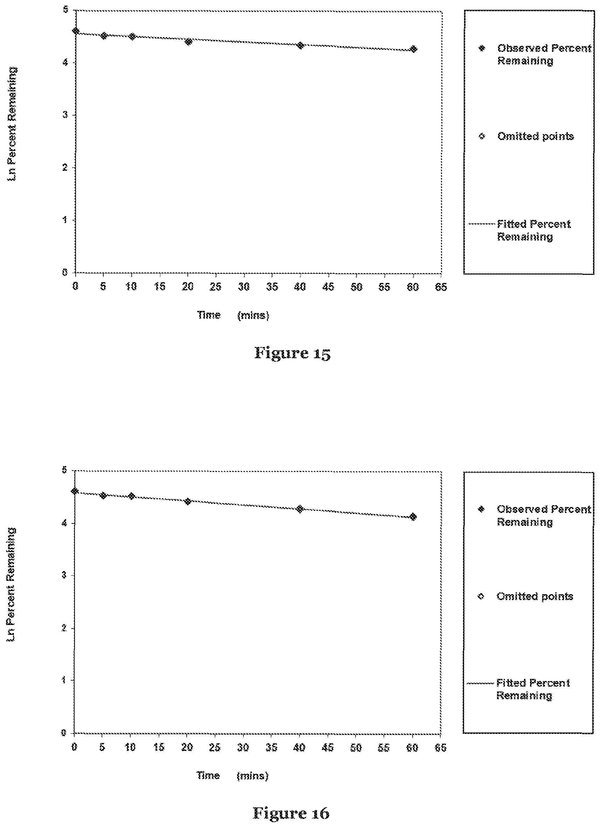

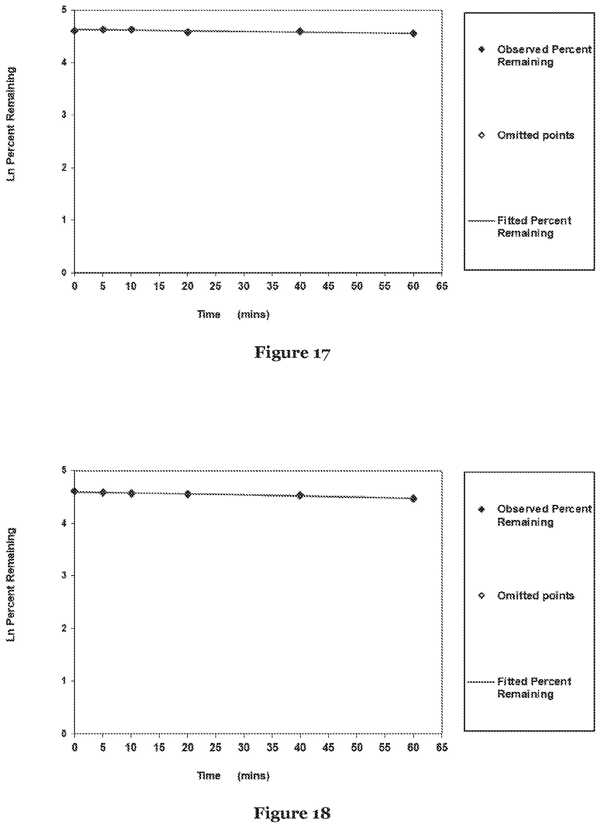

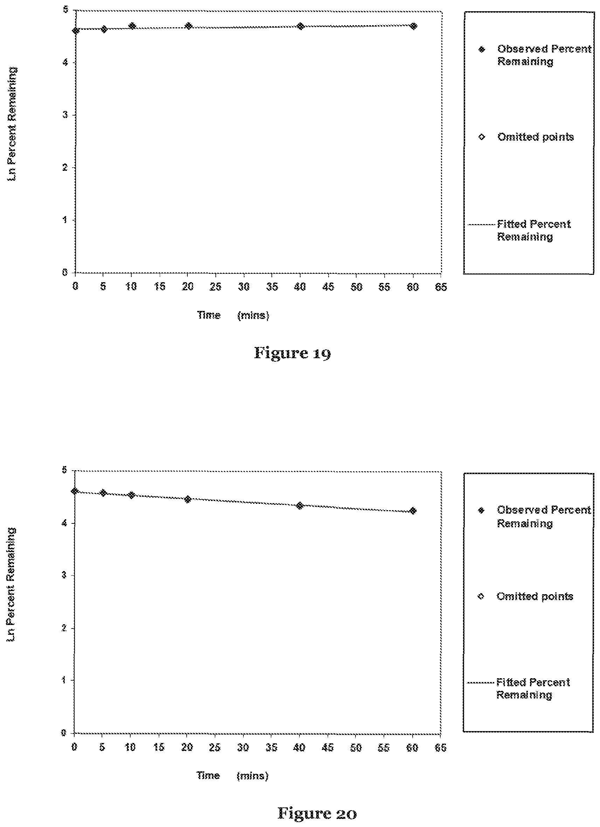

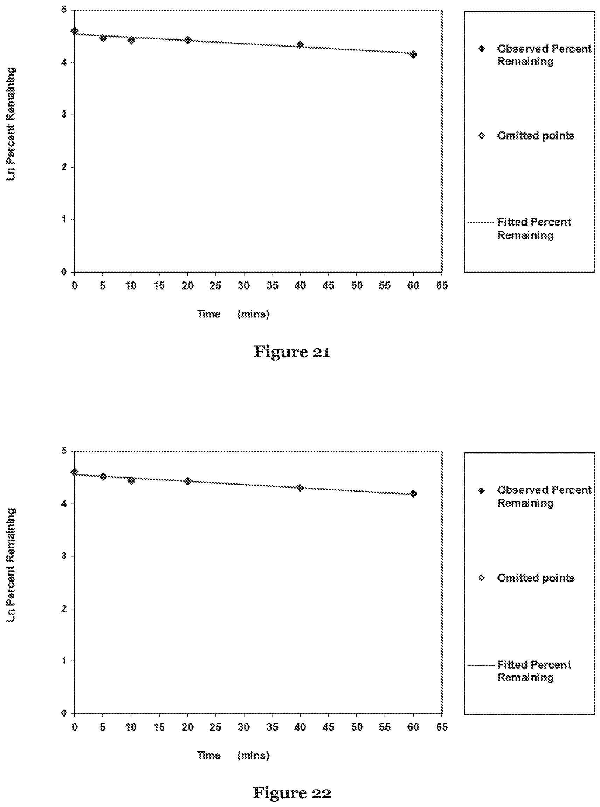

Mebendazole gave intrinsic clearance values of 12.2±0.697, 12.1±1.98 and 12.3±1.37 µL/min/10⁶ cells in the presence of 30, 100 and 300 µM cimetidine respectively and 2.33±0.764, 3.90±0.375 and -2.80±1.50 µL/min/10⁶ cells in the presence of 10, 30 and 100 µM thiabendazole respectively in human cryopreserved hepatocytes. The results are shown in Table 8 and Table 9 and in Figures 16 to 22.

**Table 8: Hepatocyte stability data for ethoxycoumarin (positive control) and for the control compounds**

| **Compound** | **Inhibitor** | **CLᵢₙₜ (µL/min/10⁶ cells)** | **SE CLᵢₙₜ** | **t_{½} (min)** | **n** |
|---|---|---|---|---|---|
| Verapamil | None | 90.3 | 3.52 | 15.4 | 6 |
| Umbelliferone | None | 229 | 2.59 | 6.05 | 4^{a} |
| Ethoxycoumarin | None | 105 | 4.26 | 13.2 | 6 |
| | Cimetidine (30 µM) | 99.9 | 3.66 | 13.9 | 6 |
| | Cimetidine (100 µM) | 88.8 | 2.66 | 15.6 | 6 |
| | Cimetidine (300 µM) | 59.8 | 3.29 | 23.2 | 6 |
| | Thiabendazole (10 µM) | 8.65 | 0.546 | 160 | 6 |
| | Thiabendazole (30 µM) | 5.35 | 0.830 | 259 | 6 |
| | Thiabendazole (100 µM) | 4.05 | 0.937 | 343 | 6 |

| | | | | | |
|---|---|---|---|---|---|
| n = number of time points used to fit curve; SE = Standard error of the curve fitting a 40 and 60 minute time points excluded. | | | | | |

**Table 9: Hepatocyte stability data for mebendazole**

| **Inhibitor** | **CLᵢₙₜ (µL/min/10⁶ cells)** | **SE CLᵢₙₜ** | **t_{½} (min)** | **n** |
|---|---|---|---|---|
| None | 15.0 | 0.658 | 92.4 | 6 |
| Cimetidine (30 µM) | 12.2 | 0.697 | 114 | 6 |
| Cimetidine (100 µM) | 12.1 | 1.98 | 115 | 6 |
| Cimetidine (300 µM) | 12.3 | 1.37 | 113 | 6 |
| Thiabendazole (10 µM) | 2.33 | 0.764 | 596 | 6 |
| Thiabendazole (30 µM) | 3.90 | 0.375 | 356 | 6 |
| Thiabendazole (100 µM) | -2.80 | 1.50 | -495 | 6 |

| | | | | |
|---|---|---|---|---|
| n = number of time points used to fit curve; SE = Standard error of the curve fitting | | | | |

In conclusion, metabolism of mebendazole was markedly decreased in the presence of the CYP1A2 inhibitor thiabendazole (6.4 fold (84% inhibition of intrinsic clearance)) and to a much lesser extent in the presence of cimetidine (reference compound) (1.2 fold (19% inhibition of intrinsic clearance)). Half-lives of mebendazole were up to 115 minutes in the presence of cimetidine and up to 596 minutes in the presence of thiabendazole, compared to 92.4 minutes without any inhibitor.

### Example 7: Study to Investigate the Stability of the Test Compound, Mebendazole, in Human Cryopreserved Hepatocytes in the Absence and Presence of the Inhibitors Cimetidine, Fluvoxamine or Mexiletine

The objective of this study was to determine the stability of the test compound, mebendazole, in the presence of human cryopreserved hepatocytes in the absence and presence of selected inhibitors (cimetidine, fluvoxamine or mexiletine).

Mebendazole (1 µM) was incubated with human cryopreserved hepatocytes for 60 min in the absence and presence of selected inhibitors (cimetidine, fluvoxamine or mexiletine) and samples taken at the required time points. The amount of test compound remaining at each time point was analysed by LC-MS/MS. Appropriate control compounds were also incubated.

The positive control compounds behaved as expected in the assays and all intrinsic clearance values were within acceptable limits where applicable.

Mebendazole was metabolised by cryopreserved human hepatocytes. Metabolism of mebendazole was not decreased in the presence of cimetidine. In initial experiments, metabolism of mebendazole was reduced 2.3 fold and 2.7 fold, respectively, at 30 µM fluvoxamine and 10 µM mexiletine. Further confirmatory experiments with cryopreserved human hepatocytes at a concentration range of 10 to 300 µM showed marked reduction of metabolism in the presence of fluvoxamine (up to 20.2 fold as seen at 10 µM) and mexiletine (up to 7.8 fold as seen at 30 µM). Half-lives of mebendazole were up to 1840 minutes in the presence of fluvoxamine and up to 713 minutes in the presence of mexiletine, compared to 91.4 minutes without any inhibitor.

In these hepatocyte stability investigations, Williams E media supplemented with 2 mM L-glutamine and 25 mM HEPES, test compound (final substrate concentration 1 µM; final DMSO concentration 0.25 %) and, where applicable, the selected inhibitor (final methanol concentration 0.08 %), were pre incubated at 37 °C prior to the addition of a suspension of cryopreserved hepatocytes (final cell density 0.5 × 10⁶ viable cells/mL in Williams E media supplemented with 2 mM L glutamine and 25 mM HEPES) to initiate the reaction. The final incubation volume was 500 µL. Appropriate control compounds were included, alongside appropriate vehicle control. Details of inhibitors and concentrations are shown in the Table 10 below.

**Table 10: Inhibitor and concentration conditions for human hepatocytes**

| **Study** | **Inhibitor** | **Inhibitor Concentration (µM)** |
|---|---|---|
| Initial study | None | NA |
| | Cimetidine | 30, 100, 300 |
| | Fluvoxamine | 3, 10, 30 |
| | Mexiletine | 10, 30, 100 |
| Confirmatory study | None | NA |
| | Fluvoxamine | 10, 30, 100, 300 |
| | Mexiletine | 10, 30, 100, 300 |

The reactions were stopped by transferring 50 µL of incubate to 100 µL acetonitrile containing internal standard at the appropriate time points (0, 5, 10, 20, 40 and 60 min). The termination plates were centrifuged at 2500 rpm at 4 °C for 30 min to precipitate the protein.

Two control compounds were included in the assays and if the values for these compounds were not within the specified limits (where available) the results were rejected and the experiment repeated.

In the initial study, the positive control compounds behaved as expected in the assay and all intrinsic clearance values were within acceptable limits. Mebendazole gave an intrinsic clearance value of 12.1 ±0.601 µL/min/10⁶ cells in the absence of inhibitor in human cryopreserved hepatocytes.

Mebendazole gave intrinsic clearance values of 24.8±1.82, 18.0±1.19 and 24.8±2.36 µL/min/10⁶ cells in the presence of 30, 100 and 300 µM cimetidine (reference compound) respectively, 8.14±1.53, 10.8±2.32 and 5.37±1.75 µL/min/10⁶ cells in the presence of 3, 10 and 30 µM fluvoxamine respectively and 4.53±1.46, 10.5±1.65 and 11.6±1.04 µL/min/10⁶ cells in the presence of 10, 30 and 100 µM mexiletine respectively in human cryopreserved hepatocytes. The results are shown in Table 11.

**Table 11: Initial Study: Human hepatocyte stability data for mebendazole with and without inhibitors**

| **Inhibitor** | **CLᵢₙₜ (µL/min/10⁶ cells)** | **SE CLᵢₙₜ** | **t_{½} (min)** | **n** |
|---|---|---|---|---|
| None | 12.1 | 0.601 | 115 | 6 |
| Cimetidine (30 µM) | 24.8 | 1.82 | 55.9 | 6 |
| Cimetidine (100 µM) | 18.0 | 1.19 | 77.2 | 6 |
| Cimetidine (300 µM) | 24.8 | 2.36 | 55.9 | 6 |
| Fluvoxamine (3 µM) | 8.14 | 1.53 | 170 | 6 |
| Fluvoxamine (10 µM) | 10.8 | 2.32 | 128 | 6 |
| Fluvoxamine (30 µM) | 5.37 | 1.75 | 258 | 6 |
| Mexiletine (10 µM) | 4.53 | 1.46 | 306 | 6 |
| Mexiletine (30 µM) | 10.5 | 1.65 | 132 | 6 |
| Mexiletine (100 µM) | 11.6 | 1.04 | 120 | 6 |

| | | | | |
|---|---|---|---|---|
| n = number of time points used to fit curve; SE = Standard error of the curve fitting | | | | |

In the confirmatory study, the positive control compounds behaved as expected in the assay and all intrinsic clearance values were within acceptable limits. Mebendazole gave an intrinsic clearance value of 15.2±3.84 µL/min/10⁶ cells in the absence of inhibitor in human cryopreserved hepatocytes.

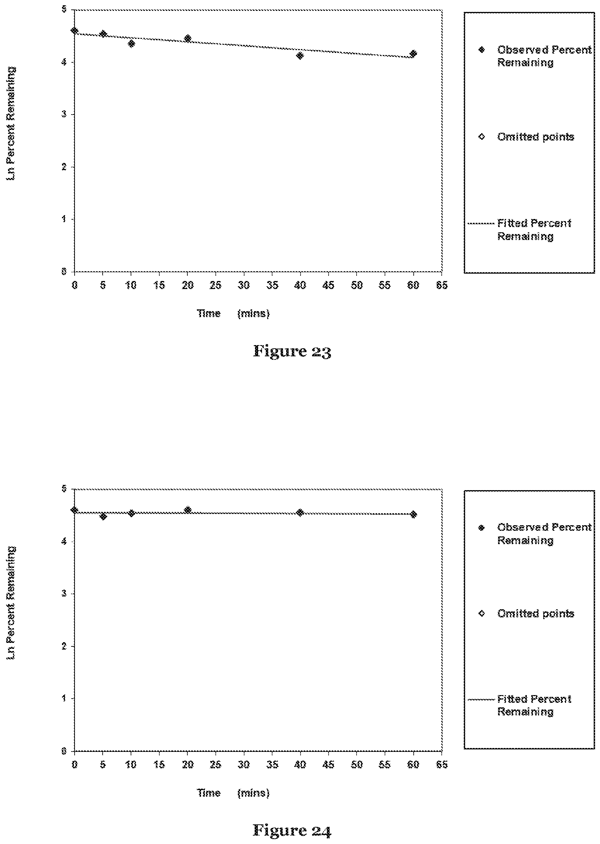

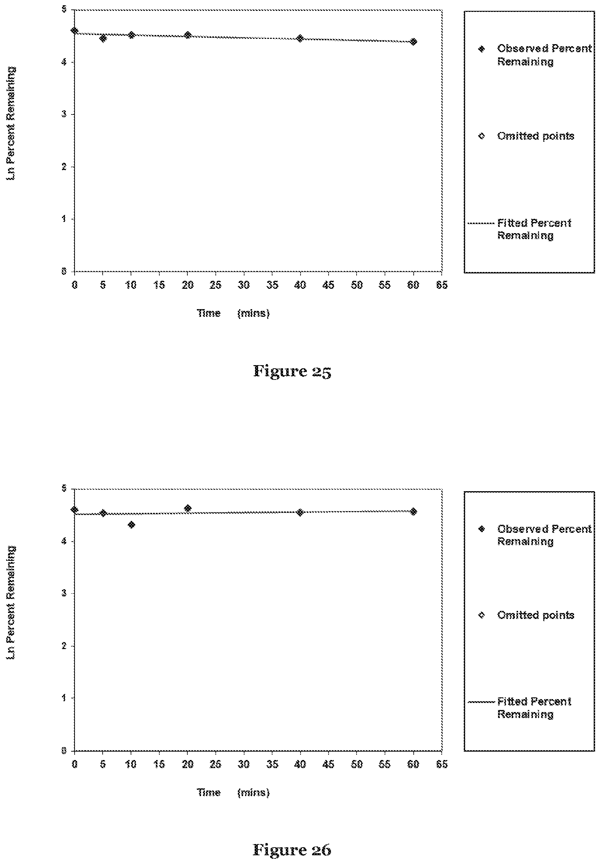

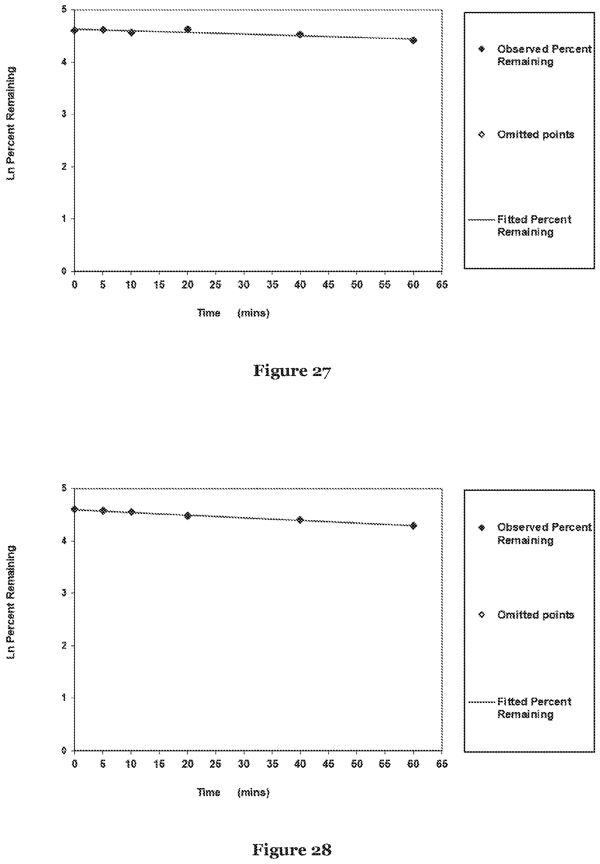

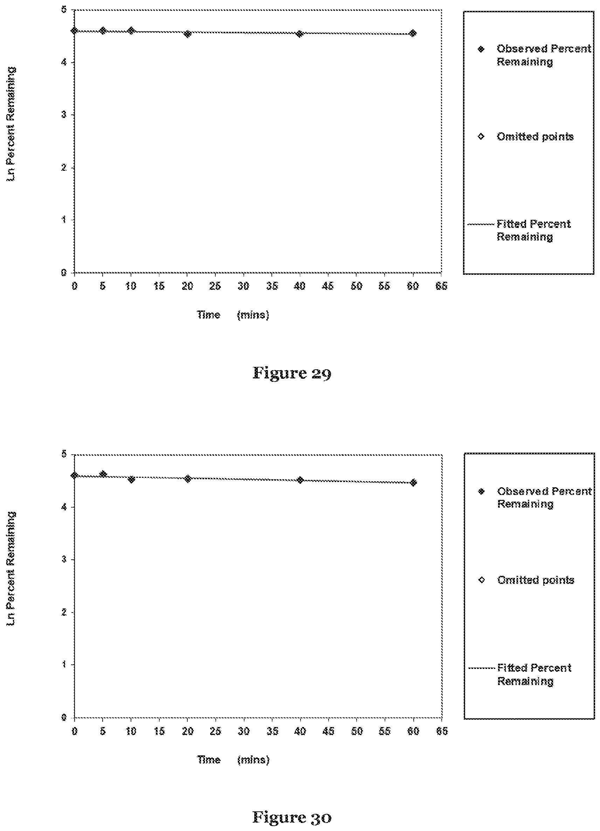

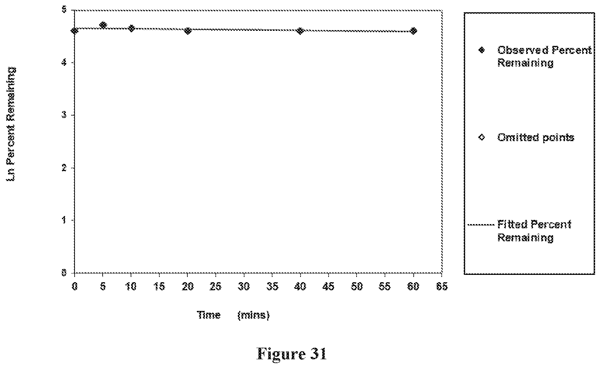

Mebendazole gave intrinsic clearance values of 0.751±2.16, 4.91±41.93, -1.99±4.70 and 6.16±1.70 µL/min/10⁶ cells in the presence of 10, 30, 100 and 300 µM fluvoxamine respectively and 10.240.458, 1.94±0.917, 4.37±1.28 and 2.08±1.80 µL/min/10⁶ cells in the presence of 10, 30, 100 and 300 µM mexiletine respectively in human cryopreserved hepatocytes. The results are shown in Table 12 and in Figures 23 to 31.

**Table 12: Confirmatory Study: Human hepatocyte stability data for mebendazole with and without inhibitors**

| **Inhibitor** | **CLᵢₙₜ (µL/min/10⁶ cells)** | **SE CLᵢₙₜ** | **t_{½} (min)** | **n** |
|---|---|---|---|---|
| None | 15.2 | 3.84 | 91.4 | 6 |
| Fluvoxamine (10 µM) | 0.751 | 2.16 | 1840 | 6 |
| Fluvoxamine (30 µM) | 4.91 | 1.93 | 282 | 6 |
| Fluvoxamine (100 µM) | -1.99 | 4.70 | -697 | 6 |
| Fluvoxamine (300 µM) | 6.16 | 1.70 | 225 | 6 |
| Mexiletine (10 µM) | 10.2 | 0.458 | 136 | 6 |
| Mexiletine (30 µM) | 1.94 | 0.917 | 713 | 6 |
| Mexiletine (100 µM) | 4.37 | 1.28 | 317 | 6 |
| Mexiletine (300 µM) | 2.08 | 1.80 | 666 | 6 |

| | | | | |
|---|---|---|---|---|
| n = number of time points used to fit curve; SE = Standard error of the curve fitting | | | | |

Metabolism of mebendazole was not decreased in the presence of cimetidine in cryopreserved human hepatocytes. Confirmatory experiments with cryopreserved human hepatocytes at a concentration range of 10 to 300 µM showed marked reduction of metabolism in the presence of fluvoxamine (up to 20.2 fold as seen at 10 µM (95% inhibition of intrinsic clearance)) and mexiletine (up to 7.8 fold as seen at 30 µM (87% inhibition of intrinsic clearance)). Half-lives of mebendazole were up to 1840 minutes in the presence of fluvoxamine and up to 713 minutes in the presence of mexiletine, compared to 91.4 minutes without any inhibitor.

### Example 8: Study to Investigate the Stability of the Test Compound, Ricobendazole (Albendazole Sulphoxide), in Human Microsomes in the Absence and Presence of the Inhibitors Cimetidine and Furafylline, and Cytochrome Reaction Phenotyping of Ricobendazole (reference compound)

The objective of this study was to determine the stability of the test compound, ricobendazole (albendazole sulphoxide), in the presence of human liver microsomes in the absence and presence of the inhibitors cimetidine and furafylline, and to determine the stability of ricobendazole in the presence of recombinant enzymes, CYP1A2, CYP2B6, CYP2C8, CYP2C9, CYP2C19, CYP2D6 and CYP3A4.

In order to optimise metabolic stability conditions, ricobendazole was incubated with human liver microsomes (1 µM at 0.5 mg/mL and 0.1 µM at 1 mg/mL) and NADPH for 45 min and samples taken at the required time points. The amount of test compound remaining at each time point was analysed by LC MS/MS. Incubation in the absence of NADPH was also performed to assess non-CYP mediated stability. Appropriate control compounds were also incubated.

For the optimisation method, the positive control compounds behaved as expected in the assays and all intrinsic clearance values were within acceptable limits.

Ricobendazole gave an intrinsic clearance value of 4.39±2.77 µL/min/mg protein when tested at 1 µM and 0.5 mg/mL protein. Ricobendazole gave an intrinsic clearance value of 6.63±1.84 µL/min/mg protein when tested at 0.1 µM and 1 mg/mL protein.

Following optimisation, human liver microsomes (final protein concentration 1 mg/mL), 0.1 M phosphate buffer pH 7.4 and NADPH (final concentration 1 mM) were pre incubated at 37 °C prior to the addition of test compound (final ricobendazole substrate concentration 0.1 µM; final DMSO concentration 0.25 %) to initiate the reaction. A minus cofactor control incubation was included for each compound tested where 0.1 M phosphate buffer pH 7.4 was added instead of NADPH (minus NADPH). Incubations were performed in the absence of inhibitor and in the presence of cimetidine (30, 100 or 300 µM) or furafylline (3, 10 or 30 µM). A control compound (phenacetin) was included (incubated without and with inhibitors as described above). All incubations were performed singularly for each test compound. Each incubation was carried out for 0, 5, 15, 30 and 45 min. The control (minus NADPH) was incubated for 45 min only. The reactions were stopped by transferring incubate into acetonitrile at the appropriate time points, in a 1:3 ratio. The termination plates were centrifuged at 3,000 rpm for 20 min at 4 °C to precipitate the protein. The amount of test compound remaining at each time point was analysed by LC MS/MS.

Relevant control compounds were assessed, ensuring intrinsic clearance values fell within the specified limits. The positive control compound behaved as expected in the assay.

Ricobendazole gave an intrinsic clearance value of 2.83±1.76 µL/min/mg protein in the absence of inhibitor.

Ricobendazole gave intrinsic clearance values of 3.15±1.24, 3.43±1.56 and 3.54±1.09 µl/min/mg protein in the presence of 30, 100 and 300 µM cimetidine respectively.

Ricobendazole gave intrinsic clearance values of 1.27±2.47, 4.31±2.48 and 2.92±1.75 µL/min/mg protein in the presence of 3, 10 and 30 µM furafylline respectively.

**Table 13: Microsomal metabolic stability ricobendazole data (inhibitors method)**

| **Inhibitor** | **Inhibitor Conc** | **CLᵢₙₜ (µL/min/mg protein)** | **SE CLᵢₙₜ** | **t_{½} (min)** | **n** |
|---|---|---|---|---|---|
| None | NA | 2.83 | 1.76 | 245 | 5 |
| Cimetidine | 30 µM | 3.15 | 1.24 | 220 | 5 |
| | 100 µM | 3.43 | 1.56 | 202 | 5 |
| | 300 µM | 3.54 | 1.09 | 196 | 5 |
| Furafylline | 3 µM | 1.27 | 2.47 | 545 | 5 |
| | 10 µM | 4.31 | 2.48 | 161 | 5 |
| | 30 µM | 2.92 | 1.75 | 237 | 5 |

| | | | | | |
|---|---|---|---|---|---|
| n = number of time points used to fit curve; SE = Standard error of the curve fitting | | | | | |

For the reaction phenotyping assay, Bactosomes^{™}, 0.1 M phosphate buffer pH 7.4 and test compound (final ricobendazole substrate concentration 1 µM; final DMSO concentration 0.25 %) were pre-incubated at 37 °C prior to the addition of NADPH (final concentration 1 mM) to initiate the reaction. Incubations were also performed using control Bactosomes^{™} (no P450 enzymes present) to reveal any non-enzymatic degradation. Control compounds known to be metabolised specifically by each P450 isoform were included. All incubations were performed singularly for each test compound. Each compound was incubated for 0, 5, 15, 30 and 45 min with each isoform. The reactions were stopped by transferring an aliquot of incubate to quench solvent at the appropriate time points, in a 1:3 ratio. The termination plates were centrifuged at 3,000 rpm for 20 min at 4 °C to precipitate the protein. The amount of test compound remaining at each time point was analysed by LC-MS/MS.

The positive control compounds behaved as expected in the assays and all half-life values were within acceptable limits. Ricobendazole gave half-life values of 240±38.1, 272±160, 618±650, 465±273 and 489±682 min in the presence of CYP1A2, CYP2C9, CYP2C19, CYP2D6, and CYP3A4 recombinant enzymes respectively. Ricobendazole was not metabolised by CYP2B6 or CYP2C8 recombinant enzymes.

**Table 14: Reaction phenotyping ricobendazole data**

| **Isoform** | **t_{½} (min)** | **SE t_{½} (min)** | **n** |
|---|---|---|---|
| 1A2 | 240 | 38.1 | 5 |
| 2B6 | -1760 | 5720 | 5 |
| 2C8 | -423 | 90.0 | 5 |
| 2C9 | 272 | 160 | 5 |
| 2C19 | 618 | 650 | 5 |
| 2D6 | 465 | 273 | 5 |
| 3A4 | 489 | 682 | 5 |

| | | | |
|---|---|---|---|
| n = number of time points used to fit curve; SE = Standard error of the curve fitting | | | |

In conclusion, ricobendazole had low turnover in human liver microsomes and little effect was observed with the addition of specific inhibitors (cimetidine and furafylline); therefore the contribution of CYP1A2 to the metabolism of ricobendazole was not conclusive. Ricobendazole showed low turnover in recombinant enzymes; therefore the contribution of specific CYP isoforms to the metabolism of ricobendazole was not conclusive, although there was some evidence of limited turnover with CYP1A2 (85% remaining at 45 min, and taking into account the half-life value of 240±38.1).

### Example 9: Two-dimensional monolayer screening for in vitro anticancer activity using neuroblastoma and sarcoma derived cell lines

Seventeen compounds, including mebendazole (Batch no. MKCC1885, Sigma-Aldrich, 100% pure), were tested in vitro across a panel of 8 selected neuroblastoma (NBXF) and 13 selected sarcoma (SXF) derived cell lines by using a 2D monolayer assay. Compounds were tested at 10 concentrations in half-log increments up to 100 µM and cells were treated for a period of 72 hours. Efficacy of compounds was assessed using CellTiter-Blue^{®} Cell Viability Assay. Anti-tumor activity is expressed as absolute and relative IC₅₀ and IC₇₀ values, calculated by non-linear regression analysis.

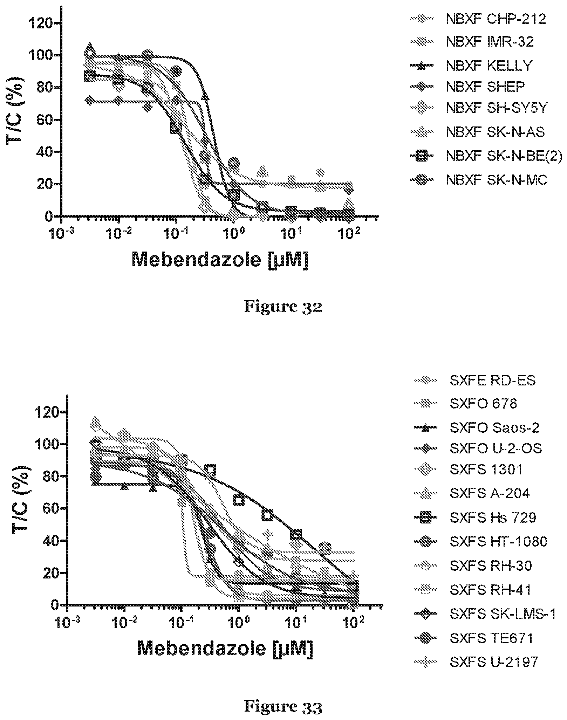

Results are shown in Tables 15 and 16 and Figures 32 and 33. By exhibiting relative and absolute geometric mean IC₅₀ values of 0.28 and 0.291 µM, respectively, mebendazole showed the highest potency across the cell lines tested.

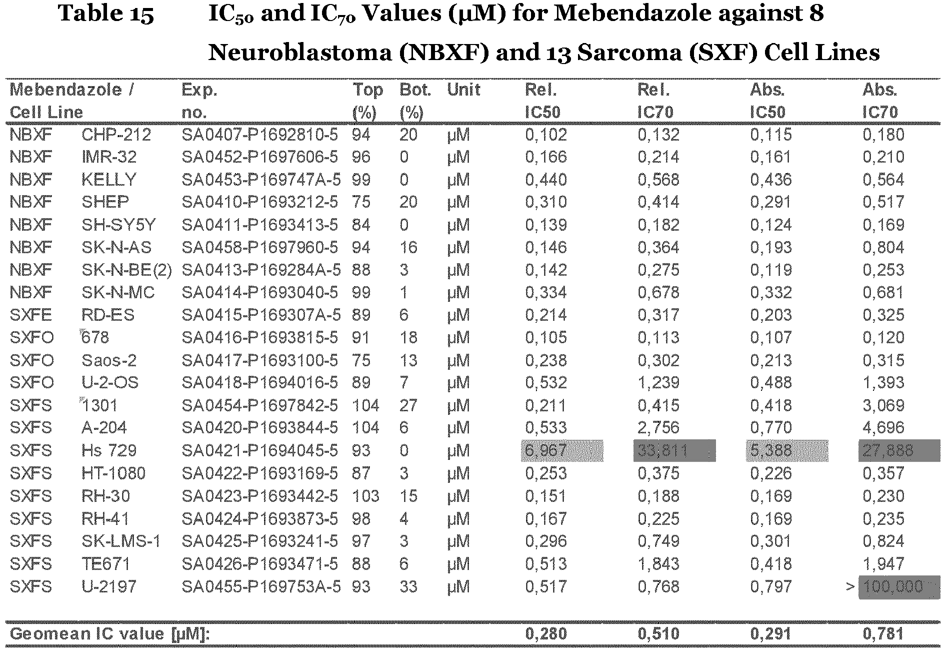

**Table 16 Test/Control Values (%) at each Mebendazole Test Concentration (µM) for 8 Neuroblastoma and 13 Sarcoma Cell Lines**

| **Mebendazole / Cell Line** | | **Exp. no.** | **Test/Control (%) at Drug Concentration [µM]** | | | | | | | | | |
|---|---|---|---|---|---|---|---|---|---|---|---|---|
| | | | **3.2E-03** | **1,0E-02** | **3,2E-0** | **1,0E-01** | **3,2E-01** | **1,0E+00** | **3,2E+00** | **1,0E+01** | **3,2E+01** | **1,0E+02** |
| NBXF | CHP-212 | SA0407-P1692810-5 | 95 | 98 | 88 | 59 | 19 | 14 | 20 | 23 | 27 | 19 |
| NBXF | IMR-32 | SA0452-P1697606-5 | 97 | 95 | 94 | 81 | 10 | 1 | 1 | 0 | -1 | -2 |
| NBXF | KELLY | SA0453-P169747A-5 | 106 | 99 | 99 | 91 | 75 | 6 | -1 | -1 | -1 | -1 |
| NBXF | SHEP | SA0410-P1693212-5 | 72 | 72 | 68 | 72 | 47 | 20 | 27 | 20 | 18 | 16 |
| NBXF | SH-SY5Y | SA0411-P1693413-5 | 94 | 81 | 78 | 63 | 6 | 1 | 1 | 0 | 0 | -1 |
| NBXF | SK-N-AS | SA0458-P1697960-5 | 89 | 91 | 84 | 62 | 36 | 31 | 28 | 21 | 19 | 8 |
| NBXF | SK-N-BE(2) | SA0413-P169284A-5 | 87 | 85 | 80 | 55 | 23 | 13 | 6 | 3 | 2 | 1 |
| NBXF | SK-N-MC | SA0414-P1693040-5 | 101 | 88 | 100 | 90 | 38 | 33 | 6 | 1 | 1 | 0 |
| SXFE | RD-ES | SA0415-P169307A-5 | 93 | 88 | 84 | 78 | 29 | 14 | 7 | 6 | 5 | 3 |
| SXFO | 678 | SA0416-P1693815-5 | 90 | 87 | 88 | 64 | 14 | 11 | 22 | 28 | 21 | 11 |
| SXFO | Saos-2 | SA0417-P1693100-5 | 77 | 74 | 73 | 73 | 29 | 19 | 20 | 11 | 12 | 6 |
| SXFO | U-2-OS | SA0418-P1694016-5 | 85 | 89 | 86 | 82 | 54 | 38 | 19 | 13 | 11 | 6 |
| SXFS | 1301 | SA0454-P1697842-5 | 101 | 106 | 98 | 84 | 55 | 35 | 33 | 38 | 36 | 8 |
| SXFS | A-204 | SA0420-P1693844-5 | 114 | 96 | 83 | 79 | 60 | 45 | 35 | 26 | 20 | 10 |
| SXFS | Hs 729 | SA0421-P1694045-5 | 93 | 91 | 89 | 90 | 84 | 65 | 56 | 44 | 35 | 12 |
| SXFS | HT-1080 | SA0422-P1693169-5 | 90 | 89 | 79 | 79 | 35 | 7 | 4 | 4 | 3 | 1 |
| SXFS | RH-30 | SA0423-P1693442-5 | 111 | 104 | 95 | 89 | 20 | 19 | 21 | 20 | 13 | 5 |
| SXFS | RH-41 | SA0424-P1693873-5 | 95 | 105 | 93 | 81 | 17 | 5 | 5 | 6 | 4 | 1 |
| SXFS | SK-LMS-1 | SA0425-P1693241-5 | 101 | 90 | 83 | 80 | 45 | 27 | 16 | 9 | 5 | 3 |
| SXFS | TE671 | SA0426-P1693471-5 | 80 | 87 | 85 | 72 | 45 | 38 | 32 | 17 | 13 | 5 |
| SXFS | U-2197 | SA0455-P169753A-5 | 93 | 93 | 93 | 91 | 79 | 42 | 44 | 37 | 36 | 18 |

| | | | | | | | | | | | | |
|---|---|---|---|---|---|---|---|---|---|---|---|---|
| NBXF neuroblastoma; SXFE Ewing sarcoma; SXFO Osteosarcoma; SXFS soft tissue sarcoma | | | | | | | | | | | | |

### Example 10: Testing Mebendazole against 12 cell lines (6 Ovarian and 6 Renal) including P-gp expressing, multidrug resistant OVXF 899L and RXF 486L PDX-derived cell lines

Mebendazole was tested in vitro across a panel of 12 tumor cell lines of ovarian and renal cancer by using a 2D monolayer assay. In this study, PDX-derived cell lines and publicly available cell lines of ovarian cancer (n=6, OVXF 1023L, OVXF 899L, A2780, OVCAR-3, OVCAR-5, SK-OV-3), and renal cancer (n=6, RXF 1183L, RXF 1220L, RXF 1781L, RXF 2282L, RXF 486L, 786-O) were used. OVXF 899L and RXF 486L are P-gp expressing, multidrug resistant cell lines.

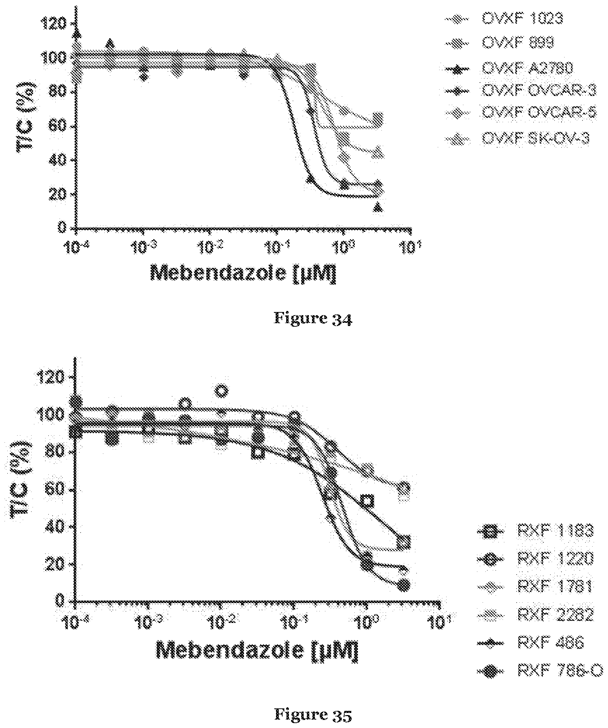

Mebendazole was tested at 10 concentrations in half-log increments up to a concentration of 3.2 µM, and cells were treated for a period of 72 h. Efficacy of compounds was assessed using CellTiter-Blue^{®} cell viability assay. Anti-tumor activity is expressed as absolute and relative IC50/70 values, calculated by 4-parameter non-linear curve fit. Results for mebendazole are shown in Tables 17 to 18 and Figures 34 and 35.

By exhibiting a mean geometric relative (absolute) IC₅₀ value of 0.587 µM (0.941 µM), mebendazole was clearly very active.

Mebendazole was most active against the ovarian cancer cell line A2780 (rel. IC₅₀ = 0.178 µM) and the renal cancer cell line RXF 486L (0.248 µM). In addition to a relative low IC₅₀ value, for both cell lines, the bottom plateau of the concentration-effect curve was below T/C = 20% in these cases.

Regarding the P-gp expressing, multidrug resistant cell lines OVXF 899L and RXF 486L, mebendazole appeared not to be a substrate for the P-gp efflux pump. Both cell lines were sensitive to mebendazole, with RXF 486L showing above-average sensitivity.

**Table 17 IC₅₀ and IC₇₀ Values (µM) for Mebendazole against 6 Ovarian Cancer (OVXF) Cell Lines and 6 Renal Cancer (RXF) Cell Lines**

| **Mono: Mebendazole Cell Line** | | **Exp. no.** | **Top (%)** | **Bot. (%)** | **Unit** | **Rel. IC50** | **Rel. IC70** | **Abs. IC50** | **Abs. IC70** |
|---|---|---|---|---|---|---|---|---|---|
| OVXF | 1023 | SA1434-P1785842-5 | 105 | 2 | µM | > 3,200 | > 3,200 | > 3,200 | > 3,200 |
| OVXF | 899 | SA1433-P178640A-5 | 95 | 57 | µM | 0,401 | 0,425 | > 3,200 | > 3,200 |
| OVXF | A2780 | SA1435-P1786600-5 | 102 | 19 | µM | 0,178 | 0,231 | 0,209 | 0,323 |
| OVXF | OVCAR-3 | SA1438-P1787002-5 | 95 | 26 | µM | 0,362 | 0,447 | 0,426 | 0,749 |
| OVXF | OVCAR-5 | SA1436-P1786801-5 | 97 | 20 | µM | 0,732 | 0,995 | 0,865 | 1,480 |
| OVXF | SK-OV-3 | SA1437-P1786617-5 | 100 | 44 | µM | 0,476 | 0,679 | 1,199 | > 3,200 |
| RXF | 1183 | SA1442-P1787019-5 | 91 | 0 | µM | 1,309 | > 3,200 | 0,925 | > 3,200 |
| RXF | 1220 | SA1444-P1786818-5 | 103 | 59 | µM | 0,398 | 0,751 | > 3,200 | > 3,200 |
| RXF | 1781 | SA1440-P1785859-5 | 96 | 28 | µM | 0,309 | 0,410 | 0,395 | 0,978 |
| RXF | 2282 | SA1441-P1785865-5 | 102 | 0 | µM | > 3,200 | > 3,200 | > 3,200 | > 3,200 |
| RXF | 486 | SA1439-P1786623-5 | 95 | 18 | µM | 0,248 | 0,362 | 0,292 | 0,545 |
| RXF | 786-O | SA1443-P1785871-5 | 94 | 9 | µM | 0,457 | 0,640 | 0,470 | 0,712 |
| **Geometric mean IC value [µM]:** | | | | | | **0,587** | **0,801** | **0,941** | **1,515** |

**Table 18 Test/Control Values (%) at each Mebendazole Test Concentration (µM) for 6 Ovarian Cancer (OVXF) Cell Lines and 6 Renal Cancer (RXF) Cell Lines**

| **Mono: Mebendazole/ Cell Line** | | **Exp. no.** | **Test/C ontrol (%) at Drug C Concentra tion [µM]** | | | | | | | | | |
|---|---|---|---|---|---|---|---|---|---|---|---|---|
| | | | **1,0 E-04** | **3,2E-04** | **1.0E-03** | **3.2E-03** | **1,0 E-02** | **3.2E-02** | **1.0E-01** | **3,2E-01** | **1.0E+00** | **3.2E+00** |
| OVXF | 1023 | SA1434-P1785842-5 | 107 | 103 | 104 | 101 | 101 | 98 | 94 | 82 | 69 | 62 |
| OVXF | 899 | SA1433-P178640A-5 | 88 | 98 | 100 | 97 | 97 | 94 | 92 | 94 | 54 | 65 |
| OVXF | A2780 | SA1435-P1786600-5 | 115 | 109 | 95 | 99 | 96 | 97 | 91 | 30 | 26 | 13 |
| OVXF | OVCAR-3 | SA1438-P1787002-5 | 97 | 103 | 89 | 93 | 97 | 90 | 95 | 69 | 27 | 26 |
| OVXF | OVCAR-5 | SA1436-P1786801-5 | 102 | 96 | 103 | 92 | 102 | 95 | 90 | 91 | 42 | 22 |
| OVXF | SK-OV-3 | SA1437-P1786617-5 | 94 | 104 | 100 | 101 | 98 | 102 | 99 | 84 | 52 | 45 |
| RXF | 1183 | SA1442-P1787019-5 | 91 | 88 | 93 | 88 | 91 | 80 | 79 | 58 | 54 | 32 |
| RXF | 1220 | SA1444-P1786818-5 | 99 | 102 | 98 | 106 | 113 | 99 | 99 | 83 | 70 | 61 |
| RXF | 1781 | SA1440-P1785859-5 | 98 | 100 | 101 | 95 | 92 | 95 | 91 | 61 | 25 | 32 |
| RXF | 1282 | SA1441-P1785865-5 | 108 | 92 | 88 | 89 | 84 | 86 | 83 | 77 | 71 | 57 |
| RXF | 486 | SA1439-P1786623-5 | 92 | 96 | 97 | 94 | 101 | 92 | 88 | 45 | 24 | 17 |
| RXF | 786-O | SA1443-P1785871-5 | 107 | 87 | 98 | 97 | 87 | 88 | 95 | 69 | 20 | 9 |

The present invention has been described with reference to various embodiments. However, the scope of the invention is not limited thereto but is defined by the following claims.

## Claims

1. A pharmaceutical composition comprising mebendazole or a pharmaceutically acceptable salt, solvate, hydrate, or *N*-oxide thereof and a strong or moderate cytochrome P450 1A2 isoenzyme (CYP1A2) inhibitor for use in a method of treatment of cancer, a non-cancer proliferative disease, a fungal disease, an inflammatory disease, or a fibrotic disease in a human.

2. A pharmaceutical composition for use according to claim 1, wherein the CYP1A2 inhibitor is furafylline, ciprofloxacin, enoxacin, fluvoxamine, zafirlukast, 8-phenyltheophylline, methoxsalen, thiabendazole or mexiletine, or a pharmaceutically acceptable salt, solvate, hydrate, or *N-*oxide thereof.

3. A pharmaceutical composition for use according to claim 2, wherein the CYP1A2 inhibitor is fluvoxamine, thiabendazole, furafylline, ciprofloxacin, or a pharmaceutically acceptable salt, solvate, hydrate, or *N*-oxide thereof.

4. A pharmaceutical composition for use according to claim 2, wherein the CYP1A2 inhibitor is ciprofloxacin, or a pharmaceutically acceptable salt, solvate, hydrate, or N-oxide thereof.

5. A pharmaceutical composition for use according to claim 2, wherein the CYP1A2 inhibitor is furafylline, or a pharmaceutically acceptable salt, solvate, hydrate, or N-oxide thereof.

6. A pharmaceutical composition for use according to claim 2, wherein the CYP1A2 inhibitor is fluvoxamine, or a pharmaceutically acceptable salt, solvate, hydrate, or N-oxide thereof.

7. A pharmaceutical composition for use according to claim 2, wherein the CYP1A2 inhibitor is thiabendazole, or a pharmaceutically acceptable salt, solvate, hydrate, or N-oxide thereof.

8. Mebendazole or a pharmaceutically acceptable salt, solvate, hydrate, or *N*-oxide thereof for use in a method of treatment of cancer, a non-cancer proliferative disease, a fungal disease, inflammatory disease, or a fibrotic disease in a human, which method of treatment comprises the simultaneous, concurrent, separate or sequential administration of a strong or moderate CYP1A2 inhibitor.

9. Mebendazole or a pharmaceutically acceptable salt, solvate, hydrate, or *N*-oxide thereof for use according to claim 8, wherein the strong or moderate CYP1A2 inhibitor is as defined in any one of claims 2 to 7.

10. A composition for use according to any one of claims 1 to 7, or mebendazole or a pharmaceutically acceptable salt, solvate, hydrate, or *N-*oxide thereof for use according to claim 8 or claim 9, wherein the method is a method of treatment of cancer or a non-cancer tumour disorder, preferably selected from Bronchial Tumors, Central Nervous System Cancer, Central Nervous System Embryonal Tumors, Carcinoma, Acute Myeloid Leukemia (AML), Carcinoid Tumor, Appendix Cancer, Astrocytomas, Chordoma, Atypical Teratoid/Rhabdoid Tumor, Sarcoma, Bladder Cancer, Thyroid Cancer, Primary Central Nervous System (CNS) Lymphoma, Extracranial Germ Cell Tumor, Esophageal Cancer, AIDS-Related Cancers, Hepatocellular (Liver) Cancer, Penile Cancer, Pleuropulmonary Blastoma, Gallbladder Cancer, Rhabdomyosarcoma, Waldenström Macroglobulinemia, Salivary Gland Cancer, Central Nervous System Germ Cell Tumors, Plasma Cell Neoplasm, Lip and Oral Cavity Cancer, Testicular Cancer, Extrahepatic Bile Duct Cancer, Ductal Carcinoma In Situ (DCIS), Nasopharyngeal Cancer, Nasal Cavity and Paranasal Sinus Cancer, Bone Cancer, Breast Cancer, Glioma, Hairy Cell Leukemia, Langerhans Cell Histiocytosis, Oral Cancer, Ependymoma, Cutaneous T-Cell Lymphoma, Gestational Trophoblastic Disease, Eye Cancer, Kaposi Sarcoma, Extragonadal Germ Cell Tumor, Gastric (Stomach) Cancer, Gastrointestinal Stromal Tumors (GIST), Papillomatosis, Small Intestine Cancer, Brain and Spinal Cord Tumors, Waldenström Macroglobulinemia, Pancreatic Cancer, Pharyngeal Cancer, Oropharyngeal Cancer, Paraganglioma, Nonmelanoma Skin Cancer, Myelodysplastic/Myeloproliferative Neoplasms, Squamous Cell Carcinoma, Malignant Fibrous Histiocytoma, Melanoma, Sezary Syndrome, Merkel Cell Carcinoma, Pituitary Tumor, Malignant Fibrous Histiocytoma of Bone and Osteosarcoma, Ovarian Cancer, Parathyroid Cancer, Skin Cancer, Mycosis Fungoides, Germ Cell Tumor, Fallopian Tube Cancer, Intraocular Melanoma, Leukemia, Pancreatic Neuroendocrine Tumors (Islet Cell Tumors), Endometrial Cancer, Lymphoma, Prostate Cancer, Renal Pelvis and Ureter Cancer, Osteosarcoma (Bone Cancer), Non-Hodgkin Lymphoma, Non-Small Cell Lung Cancer, Basal Cell Carcinoma, Laryngeal Cancer, Multiple Myeloma/Plasma Cell Neoplasm, Vaginal Cancer, Squamous Neck Cancer, Multiple Myeloma, Midline Tract Carcinoma Involving NUT Gene, Head and Neck Cancer, Heart Cancer, Intraocular (Eye), Renal Cell (Kidney) Cancer, Malignant Fibrous Histiocytoma of Bone, Liver Cancer, Rectal Cancer, Colon Cancer, Malignant Mesothelioma, Low Malignant Potential Tumor, Mouth Cancer, Soft Tissue Sarcoma, Hypopharyngeal Cancer, Wilms Tumor, Epithelial Cancer, Ewing Sarcoma Family of Tumors, Acute Lymphoblastic Leukemia (ALL), Retinoblastoma, Hodgkin Lymphoma, Brain Tumor, Esthesioneuroblastoma, Embryonal Tumors, Cervical Cancer, Chronic Myeloproliferative Neoplasms, Pancreatic Neuroendocrine Tumors, Ureter and Renal Pelvis Cancer, Anal Cancer, Urethral Cancer, Brain Stem cancer, Vulvar Cancer, Chronic Lymphocytic Leukemia (CLL), Uterine Sarcoma, Stomach (Gastric) Cancer, Brain Stem Glioma, Multiple Endocrine Neoplasia Syndromes, Myelodysplastic Syndromes, Craniopharyngioma, Small Cell Lung Cancer, Lip and Oral Cavity Cancer, Cutaneous T-Cell Lymphoma, Neuroblastoma, Acute Lymphoblastic Leukemia (ALL), Langerhans Cell Histiocytosis, Breast Cancer, Gastrointestinal Carcinoid Tumor, Paranasal Sinus and Nasal Cavity Cancer, Pheochromocytoma, Metastatic Squamous Neck Cancer with Occult Primary, Male Breast Cancer, Kidney (Renal) Cancer, Lung Cancer, Islet Cell Tumors, Extrahepatic Bile Duct Cancer, Endometrial Uterine Cancer, Chronic Myeloproliferative Neoplasms, Transitional Cell Cancer of the Renal Pelvis and Ureter, Thymoma and Thymic Carcinoma, Throat Cancer, Ewing Sarcoma, Chronic Myelogenous Leukemia (CML), Colorectal Cancer, Colon Cancer, Cardiac (Heart) Tumors, Burkitt Lymphoma, Carcinoma of Unknown Primary, Central Nervous System Atypical Teratoid/Rhabdoid Tumor, Childhood Cancers, and Non-Hodgkin Lymphoma, Adrenocortical Carcinoma, Adrenocortical Adenocarcinoma, Adrenocortical Adenoma, or P-gp expressing Multidrug Resistant Tumour, more preferably selected from Sarcoma, Ovarian Cancer, Kidney (Renal) Cancer, Melanoma, Colorectal Cancer, Colon Cancer, Lung Cancer, Brain Cancer, Adrenocortical Adenocarcinoma, Adrenocortical Carcinoma, Adrenocortical Adenoma, P-gp expressing Multidrug Resistant Tumour, and Neuroblastoma.

11. A composition for use according to any one of claims 1 to 7, or mebendazole or a pharmaceutically acceptable salt, solvate, hydrate, or *N*-oxide thereof for use according to claim 8 or claim 9, wherein the method is a method of treatment of a proliferative disease, preferably a solid tumour disorder (including cancer and non-cancer disorders), or a haematological cancer.

12. A composition for use according to any one of claims 1 to 7, or mebendazole or a pharmaceutically acceptable salt, solvate, hydrate, or *N*-oxide thereof for use according to claim 8 or claim 9, wherein the method is a method of treatment of an inflammatory disease or liver cirrhosis, preferably gout, liver cirrhosis, scleroderma, psoriasis, or endometriosis.

13. A composition for use according to any one of claims 1 to 7, or mebendazole or a pharmaceutically acceptable salt, solvate, hydrate, or *N-*oxide thereof for use according to claim 8 or claim 9, wherein the method is a method of treatment of a fungal disease, preferably selected from Cryptococcus neoformans, Encephalitozoon spp., and Enterocytozoon bieneusi.

14. A pharmaceutical composition comprising mebendazole or a pharmaceutically acceptable salt, solvate, hydrate, or *N*-oxide thereof and fluvoxamine, or a pharmaceutically acceptable salt, solvate, hydrate, or *N-*oxide thereof.

15. Mebendazole or a pharmaceutically acceptable salt, solvate, hydrate, or *N*-oxide thereof in combination with fluvoxamine, or a pharmaceutically acceptable salt, solvate, hydrate, or N-oxide thereof for use in a method of treatment of a human by therapy, which method comprises the simultaneous, concurrent, separate or sequential administration of mebendazole or a pharmaceutically acceptable salt, solvate, hydrate, or N oxide thereof and fluvoxamine, or a pharmaceutically acceptable salt, solvate, hydrate, or *N*-oxide thereof.

## Patentansprüche

1. Pharmazeutische Zusammensetzung, die Folgendes umfasst: Mebendazol oder ein pharmazeutisch akzeptables Salz, Solvat, Hydrat oder N-Oxid davon und einen starken oder mäßigen Cytochrom P450 1A2 Isoenzym- (CYP1A2) Inhibitor zur Verwendung in einem Verfahren zur Behandlung von Krebs, einer nicht krebsbedingten proliferativen Erkrankung, einer Pilzerkrankung, einer entzündlichen Erkrankung oder einer fibrotischen Erkrankung bei einem Menschen.

2. Pharmazeutische Zusammensetzung zur Verwendung nach Anspruch 1, wobei der CYP1A2-Inhibitor Furafyllin, Ciprofloxacin, Enoxacin, Fluvoxamin, Zafirlukast, 8-Phenyltheophyllin, Methoxsalen, Thiabendazol oder Mexiletin oder ein pharmazeutisch akzeptables Salz, Solvat, Hydrat oder N-Oxid davon ist.

3. Pharmazeutische Zusammensetzung zur Verwendung nach Anspruch 2, wobei der CYP1A2-Inhibitor Fluvoxamin, Thiabendazol, Furafyllin, Ciprofloxacin oder ein pharmazeutisch akzeptables Salz, Solvat, Hydrat oder N-Oxid davon ist.

4. Pharmazeutische Zusammensetzung zur Verwendung nach Anspruch 2, wobei der CYP1A2-Inhibitor Ciprofloxacin oder ein pharmazeutisch akzeptables Salz, Solvat, Hydrat oder N-Oxid davon ist.

5. Pharmazeutische Zusammensetzung zur Verwendung nach Anspruch 2, wobei der CYP1A2-Inhibitor Furafyllin oder ein pharmazeutisch akzeptables Salz, Solvat, Hydrat oder N-Oxid davon ist.

6. Pharmazeutische Zusammensetzung zur Verwendung nach Anspruch 2, wobei der CYP1A2-Inhibitor Fluvoxamin oder ein pharmazeutisch akzeptables Salz, Solvat, Hydrat oder N-Oxid davon ist.

7. Pharmazeutische Zusammensetzung zur Verwendung nach Anspruch 2, wobei der CYP1A2-Inhibitor Thiabendazol oder ein pharmazeutisch akzeptables Salz, Solvat, Hydrat oder N-Oxid davon ist.

8. Mebendazol oder ein pharmazeutisch akzeptables Salz, Solvat, Hydrat oder N-Oxid davon zur Verwendung in einem Verfahren zur Behandlung von Krebs, einer nicht krebsartigen proliferativen Erkrankung, einer Pilzerkrankung, einer entzündlichen Erkrankung oder einer fibrotischen Erkrankung bei einem Menschen, wobei das Behandlungsverfahren die gleichzeitige, begleitende, getrennte oder aufeinanderfolgende Verabreichung eines starken oder moderaten CYP1A2-Inhibitors umfasst.

9. Mebendazol oder pharmazeutisch akzeptables Salz, Solvat, Hydrat oder N-Oxid davon zur Verwendung nach Anspruch 8, wobei der starke oder moderate CYP1A2-Inhibitor wie in einem der Ansprüche 2 bis 7 definiert ist.

10. Zusammensetzung zur Verwendung nach einem der Ansprüche 1 bis 7 oder Mebendazol oder pharmazeutisch akzeptables Salz, Solvat, Hydrat oder N-Oxid davon zur Verwendung nach Anspruch 8 oder Anspruch 9, wobei das Verfahren ein Verfahren zur Behandlung von Krebs oder einer nicht krebsartigen Tumorerkrankung ist, vorzugsweise ausgewählt aus Bronchialtumoren, Krebs des zentralen Nervensystems, embryonalen Tumoren des zentralen Nervensystems, Karzinom, akuter myeloischer Leukämie (AML), karzinoidem Tumor, Blinddarmkrebs, Astrozytomen, Chordom, atypischem teratoidem/rhabdoidem Tumor, Sarkom, Blasenkrebs, Schilddrüsenkrebs, primärem Lymphom des zentralen Nervensystems (ZNS), extrakraniellem Keimzelltumor, Speiseröhrenkrebs, AIDS-assoziierten Krebserkrankungen, Leberkrebs, Peniskrebs, Pleuropulmonales Blastom, Gallenblasenkrebs, Rhabdomyosarkom, Waldenstrom-Makroglobulinämie, Speicheldrüsenkrebs, Keimzelltumoren des Zentralnervensystems, Plasmazellneoplasma, Lippen- und Mundhöhlenkrebs, Hodenkrebs, extrahepatischem Gallengangskrebs, Ductal Carcinoma In Situ (DCIS), Nasopharynxkrebs, Nasenhöhlen- und Nasennebenhöhlenkrebs, Knochenkrebs, Brustkrebs, Gliom, Haarzell-Leukämie, Langerhans-Zell-Histiozytose, Mundhöhlenkrebs, Ependymom, kutanem T-Zell-Lymphom, trophoblastischer Gestationskrankheit, Augenkrebs, Kaposi-Sarkom, extragonadalem Keimzelltumor, Magenkrebs, gastrointestinalen Stromatumoren (GIST), Papillomatose, Dünndarmkrebs, Hirn- und Rückenmarkstumoren, Waldenstrom-Makroglobulinämie, Bauchspeicheldrüsenkrebs, Rachenkrebs, Oropharynxkrebs, Paragangliom, Nichtmelanom-Hautkrebs, myelodysplastischen/myeloproliferativen Neoplasmen, Plattenepithelkarzinom, malignem fibrösem Histiozytom, Melanom, Sezary-Syndrom, Merkelzellkarzinom, Hypophysentumor, bösartigem fibrösem Histiozytom des Knochens und Osteosarkom, Eierstockkrebs, Nebenschilddrüsenkrebs, Hautkrebs, Mycosis fungoides, Keimzelltumor, Eileiterkrebs, intraokularem Melanom, Leukämie, neuroendokrinen Tumoren der Bauchspeicheldrüse (Inselzelltumore), Endometriumkrebs, Lymphom, Prostatakrebs, Nierenbecken- und Harnleiterkrebs, Osteosarkom (Knochenkrebs), Non-Hodgkin-Lymphom, nicht-kleinzelligem Lungenkarzinom, Basalzellkarzinom, Kehlkopfkrebs, multiplem Myelom/Plasmazellneoplasma, Vaginalkrebs, Plattenepithelkarzinom des Halses, multiplem Myelom, Karzinom des Mittellinientrakts mit Beteiligung des NUT-Gens, Kopf- und Halskrebs, Herzkrebs, Augenkrebs, Nierenzellkrebs, malignem fibrösem Histiozytom des Knochens, Leberkrebs, Rektalkrebs, Dickdarmkrebs, malignem Mesotheliom, Tumor mit geringem malignen Potenzial, Mundkrebs, Weichteilsarkom, Hypopharynxkrebs, Wilms-Tumor, Epithelkrebs, Ewing-Sarkom-Familie von Tumoren, Akuter Lymphoblastenleukämie (ALL), Retinoblastom, Hodgkin-Lymphom, Hirntumor, Esthesioneuroblastom, embryonalen Tumoren, Gebärmutterhalskrebs, chronischen myeloproliferativen Neoplasmen, neuroendokrinen Tumoren der Bauchspeicheldrüse, Ureter- und Nierenbeckenkrebs, Analkrebs, Harnröhrenkrebs, Hirnstammkrebs, Vulvakrebs, chronischer lymphatischer Leukämie (CLL), Uterussarkom, Magenkrebs, Hirnstammgliom, multiplen endokrinen Neoplasie-Syndromen, myelodysplastischen Syndromen, Kraniopharyngiom, kleinzelligem Lungenkrebs, Lippen- und Mundhöhlenkrebs, kutanem T-Zell-Lymphom, Neuroblastom, Akute Lymphoblastenleukämie (ALL), Langerhans-Zell-Histiozytose, Brustkrebs, gastrointestinalem Karzinoidtumor, Nasennebenhöhlen- und Nasenhöhlenkrebs, Phäochromozytom, metastasiertem Plattenepithelkarzinom-Halskrebs mit okkultem Primärtumor, männlichem Brustkrebs, Nierenkrebs, Lungenkrebs, Inselzelltumoren, extrahepatischem Gallengangskrebs, Gebärmutterkrebs, chronischen myeloproliferativen Neoplasmen, Übergangszellkrebs des Nierenbeckens und des Harnleiters, Thymom und Thymuskarzinom, Kehlkopfkrebs, Ewing-Sarkom, chronischer myeloischer Leukämie (CML), Darmkrebs, Dickdarmkrebs, Herztumoren, Burkitt-Lymphom, Karzinom unbekannter Herkunft, atypischem teratoidem/rhabdoidem Tumor des Zentralnervensystems, Krebserkrankungen im Kindesalter und Non-Hodgkin-Lymphom, Nebennierenrindenkarzinom, Nebennierenrinden-Adenokarzinom, Nebennierenrinden-Adenom oder P-gp exprimierendem multiresistenter Tumor, vorzugsweise ausgewählt aus Sarkom, Eierstockkrebs, Nierenkrebs, Melanom, Darmkrebs, Lungenkrebs, Hirntumor, Nebennierenrindenadenokarzinom, Nebennierenrindenkarzinom, Nebennierenrinden-Adenom, P-gp exprimierendem multiresistentem Tumor und Neuroblastom.

11. Zusammensetzung zur Verwendung nach einem der Ansprüche 1 bis 7 oder Mebendazol oder pharmazeutisch akzeptables Salz, Solvat, Hydrat oder N-Oxid davon zur Verwendung nach Anspruch 8 oder Anspruch 9, wobei das Verfahren ein Verfahren zur Behandlung einer proliferativen Erkrankung, vorzugsweise einer soliden Tumorerkrankung (einschließlich Krebs und Nicht-Krebs-Erkrankungen) oder eines hämatologischen Krebses ist.

12. Zusammensetzung zur Verwendung nach einem der Ansprüche 1 bis 7 oder Mebendazol oder pharmazeutisch akzeptables Salz, Solvat, Hydrat oder N-Oxid davon zur Verwendung nach Anspruch 8 oder Anspruch 9, wobei das Verfahren ein Verfahren zur Behandlung einer entzündlichen Erkrankung oder Leberzirrhose, vorzugsweise Gicht, Leberzirrhose, Sklerodermie, Psoriasis oder Endometriose ist.

13. Zusammensetzung zur Verwendung nach einem der Ansprüche 1 bis 7 oder Mebendazol oder pharmazeutisch akzeptables Salz, Solvat, Hydrat oder N-Oxid davon zur Verwendung nach Anspruch 8 oder Anspruch 9, wobei das Verfahren ein Verfahren zur Behandlung einer Pilzerkrankung ist, vorzugsweise ausgewählt aus Cryptococcus neoformans, Encephalitozoon spp. und Enterocytozoon bieneusi.

14. Pharmazeutische Zusammensetzung, die Mebendazol oder ein pharmazeutisch akzeptables Salz, Solvat, Hydrat oder N-Oxid davon und Fluvoxamin oder ein pharmazeutisch akzeptables Salz, Solvat, Hydrat oder N-Oxid davon enthält.

15. Mebendazol oder ein pharmazeutisch akzeptables Salz, Solvat, Hydrat oder N-Oxid davon in Kombination mit Fluvoxamin oder einem pharmazeutisch akzeptablen Salz, Solvat, Hydrat oder N-Oxid davon zur Verwendung in einem Verfahren zur Behandlung eines Menschen durch Therapie, wobei das Verfahren die gleichzeitige, begleitende, getrennte oder aufeinanderfolgende Verabreichung von Mebendazol oder einem pharmazeutisch akzeptablen Salz, Solvat, Hydrat oder N-Oxid davon und Fluvoxamin oder einem pharmazeutisch akzeptablen Salz, Solvat, Hydrat oder N-Oxid davon umfasst.

## Revendications

1. Composition pharmaceutique, comprenant mébendazole ou un sel pharmaceutiquement acceptable, solvate, hydrate, ou N-oxyde de celui-ci et un inhibiteur fort ou modéré d'isoenzyme du cytochrome P450 1A2 (CYP1A2) pour l'utilisation dans un procédé de traitement du cancer, d'une maladie proliférative non cancéreuse, d'une maladie fongique, d'une maladie inflammatoire, ou d'une maladie fibrotique chez un humain.

2. Composition pharmaceutique pour l'utilisation selon la revendication 1, dans laquelle l'inhibiteur de CYP1A2 est furafylline, ciprofloxacine, énoxacine, fluvoxamine, zafirlukast, 8-phénylthéophylline, méthoxsalène, thiabendazole ou méxilétine, ou un sel pharmaceutiquement acceptable, solvate, hydrate, ou N-oxyde de ceux-ci.

3. Composition pharmaceutique pour l'utilisation selon la revendication 2, dans laquelle l'inhibiteur de CYP1A2 est fluvoxamine, thiabendazole, furafylline, ciprofloxacine, ou un sel pharmaceutiquement acceptable, solvate, hydrate, ou N-oxyde de ceux-ci.

4. Composition pharmaceutique pour l'utilisation selon la revendication 2, dans laquelle l'inhibiteur de CYP1A2 est ciprofloxacine, ou un sel pharmaceutiquement acceptable, solvate, hydrate, ou N-oxyde de celle-ci.

5. Composition pharmaceutique pour l'utilisation selon la revendication 2, dans laquelle l'inhibiteur de CYP1A2 est furafylline, ou un sel pharmaceutiquement acceptable, solvate, hydrate, ou N-oxyde de celle-ci.

6. Composition pharmaceutique pour l'utilisation selon la revendication 2, dans laquelle l'inhibiteur de CYP1A2 est fluvoxamine, ou un sel pharmaceutiquement acceptable, solvate, hydrate, ou N-oxyde de celle-ci.

7. Composition pharmaceutique pour l'utilisation selon la revendication 2, dans laquelle l'inhibiteur de CYP1A2 est thiabendazole, ou un sel pharmaceutiquement acceptable, solvate, hydrate, ou *N*-oxyde de celui-ci.

8. Mébendazole ou sel pharmaceutiquement acceptable, solvate, hydrate, ou *N-*oxyde de celui-ci, pour l'utilisation dans un procédé de traitement du cancer, d'une maladie proliférative non cancéreuse, d'une maladie fongique, d'une maladie inflammatoire, ou d'une maladie fibrotique chez un humain, lequel procédé de traitement comprend l'administration simultanée, concomitante, séparée ou séquentielle d'un inhibiteur fort ou modéré de CYP1A2.

9. Mébendazole ou sel pharmaceutiquement acceptable, solvate, hydrate, ou *N-*oxyde de celui-ci, pour l'utilisation selon la revendication 8, dans lequel l'inhibiteur fort ou modéré de CYP1A2 est tel que défini dans l'une quelconque des revendications 2 à 7.

10. Composition pour l'utilisation selon l'une quelconque des revendications 1 à 7, ou mébendazole ou sel pharmaceutiquement acceptable, solvate, hydrate, ou *N-*oxyde de celui-ci, pour l'utilisation selon la revendication 8 ou la revendication 9, dans laquelle/lequel le procédé est un procédé de traitement du cancer ou d'un trouble tumoral non cancéreux, de préférence sélectionné parmi : tumeurs bronchiales, cancer du système nerveux central, tumeurs embryonnaires du système nerveux central, carcinome, leucémie myéloïde aiguë (LMA), tumeur carcinoïde, cancer de l'appendice, astrocytomes, chordome, tumeur tératoïde/rhabdoïde atypique, sarcome, cancer de la vessie, cancer de la thyroïde, lymphome du système nerveux central (SNC) primitif, tumeur germinale extracrânienne, cancer oesophagien, cancers liés au SIDA, cancer hépatocellulaire (foie), cancer pénien, blastome pleuropulmonaire, cancer de la vésicule biliaire, rhabdomyosarcome, macroglobulinémie de Waldenström, cancer des glandes salivaires, tumeurs germinales du système nerveux central, néoplasme des cellules plasmatiques, cancer de la lèvre et de la cavité buccale, cancer testiculaire, cancer de la voie biliaire extrahépatique, carcinome canalaire in situ (CCIS), cancer nasopharyngien, cancer de la cavité nasale et du sinus paranasal, cancer des os, cancer du sein, gliome, leucémie à tricholeucocytes, histiocytose des cellules de Langerhans, cancer buccal, épendymome, lymphome T cutané, maladie trophoblastique gestationnelle, cancer de l'oeil, sarcome de Kaposi, tumeur germinale extragonadique, cancer gastrique (estomac), tumeurs stromales gastrointestinales (GIST), papillomatose, cancer de l'intestin grêle, tumeurs du cerveau et de la moelle épinière, macroglobulinémie de Waldenström, cancer pancréatique, cancer pharyngien, cancer oropharyngien, paragangliome, cancer de la peau non mélanome, néoplasmes myélodysplasiques/ myéloprolifératifs, carcinome cellulaire squameux, histiocytome fibreux malin, mélanome, syndrome de Sézary, carcinome à cellules de Merkel, tumeur pituitaire, histiocytome fibreux malin des os et ostéosarcome, cancer ovarien, cancer de la parathyroïde, cancer de la peau, mycose fongoïde, tumeur germinale, cancer des trompes de Fallope, mélanome intraoculaire, leucémie, tumeurs neuroendocrines pancréatiques (tumeurs endocrines), cancer endométrial, lymphome, cancer de la prostate, cancer du pelvis rénal et de l'uretère, ostéosarcome (cancer des os), lymphome non-hodgkinien, cancer du poumon non à petites cellules, carcinome des cellules basales, cancer laryngien, myélome multiple/néoplasme des cellules plasmatiques, cancer vaginal, cancer du cou squameux, myélome multiple, carcinome de la ligne médiane NUT, cancer de la tête et du cou, cancer du coeur, cancer intraoculaire (oeil), cancer des cellules rénale (rein), histiocytome fibreux malin des os, cancer du foie, cancer rectal, cancer du côlon, mésothéliome malin, tumeur de faible potentielle malin, cancer de la bouche, sarcome des tissus mous, cancer hypopharyngien, tumeur de Wilms, cancer épithélial, famille de tumeurs du sarcome de Ewing, leucémie lymphoblasique aiguë (LLA), rétinoblastome, lymphome de Hodgkin, tumeur du cerveau, esthésioneuroblastome, tumeurs embryonnaires, cancer cervical, néoplasmes myéloprolifératifs chroniques, tumeurs neuroendocrines pancréatiques, cancer de l'uretère et du pelvis rénal, cancer anal, cancer urétral, cancer du tronc cérébral, cancer vulvaire, leucémie lymphocytique chronique (LLC), sarcome utérin, cancer de l'estomac (gastrique), gliome du tronc cérébral, syndromes de néoplasie endocrinienne multiple, syndromes myélodysplasiques, crâniopharyngiome, cancer du poumon à petites cellules, cancer de la lèvre et de la cavité buccale, lymphome T cutané, neuroblastome, leucémie lymphoblasique aiguë (LLA), histiocytose des cellules de Langerhans, cancer du sein, tumeur carcinoïde gastrointestinale, cancer du sinus paranasal et de la cavité nasale, phéochromocytome, cancer du cou squameux métastasique à primaire occulte, cancer du sein mâle, cancer du rein (rénal), cancer du poumon, tumeurs endocrines, cancer de la voie biliaire extrahépatique, cancer utérin endométrial, néoplasmes myéloprolifératifs chroniques, cancer à cellules transitionnelles du pelvis rénal et de l'uretère, thymome et carcinome thymique, cancer de la gorge, sarcome de Ewing, leucémie myélogène chronique (LMC), cancer colorectal, cancer du côlon, tumeurs cardiaques (coeur), lymphome de Burkitt, carcinome primitif inconnu, tumeur tératoïde/rhabdoïde atypique du système nerveux central, cancers de l'enfant, et lymphome non-hodgkinien, carcinome adrénocortical, adénocarcinome adrénocortical, adénome adrénocortical, ou tumeur à résistance multimédicamenteuse à expression de P-gp, de manière davantage préférée sélectionné parmi : sarcome, cancer ovarien, cancer du rein (rénal), mélanome, cancer colorectal, cancer du côlon, cancer du poumon, cancer du cerveau, adénocarcinome adrénocortical, carcinome adrénocortical, adénome adrénocortical, tumeur à résistance multimédicamenteuse à expression de P-gp, et neuroblastome.

11. Composition pour l'utilisation selon l'une quelconque des revendications 1 à 7, ou mébendazole ou sel pharmaceutiquement acceptable, solvate, hydrate, ou *N-*oxyde de celui-ci pour l'utilisation selon la revendication 8 ou la revendication 9, dans laquelle/lequel le procédé est un procédé de traitement d'une maladie proliférative, de préférence un trouble à tumeur solide (y compris des troubles cancéreux et non cancéreux), ou un cancer hématologique.

12. Composition pour l'utilisation selon l'une quelconque des revendications 1 à 7, ou mébendazole ou sel pharmaceutiquement acceptable, solvate, hydrate, ou *N-*oxyde de celui-ci pour l'utilisation selon la revendication 8 ou la revendication 9, dans laquelle/lequel le procédé est un procédé de traitement d'une maladie inflammatoire ou cirrhose hépatique, de préférence de la goutte, cirrhose hépatique, sclérodermie, du psoriasis, ou de l'endométriose.

13. Composition pour l'utilisation selon l'une quelconque des revendications 1 à 7, ou mébendazole ou sel pharmaceutiquement acceptable, solvate, hydrate, ou *N-*oxyde de celui-ci pour l'utilisation selon la revendication 8 ou la revendication 9, dans laquelle/lequel le procédé est un procédé de traitement d'une maladie fongique, de préférence sélectionnée parmi Cryptococcus neoformans, Encephalitozoon spp., et Enterocytozoon bieneusi.

14. Composition pharmaceutique, comprenant mébendazole ou un sel pharmaceutiquement acceptable, solvate, hydrate, ou *N*-oxyde de celui-ci et fluvoxamine, ou un sel pharmaceutiquement acceptable, solvate, hydrate, ou *N*-oxyde de celle-ci.

15. Mébendazole ou sel pharmaceutiquement acceptable, solvate, hydrate, ou N-oxyde de celui-ci en association avec fluvoxamine, ou un sel pharmaceutiquement acceptable, solvate, hydrate, ou N-oxyde de celle-ci pour l'utilisation dans un procédé de traitement d'un humain par thérapie, lequel procédé comprend l'administration simultanée, concomitante, séparée ou séquentielle de mébendazole ou d'un sel pharmaceutiquement acceptable, solvate, hydrate, ou *N* oxide de celui-ci et de fluvoxamine, ou d'un sel pharmaceutiquement acceptable, solvate, hydrate, ou *N-*oxyde de celle-ci.
